# EUROPEAN PATENT SPECIFICATION

(11) **EP 1 101 772 B1**
(45) Date of publication and mention of the grant of the patent: **21.01.2009**
(21) Application number: 00310121.9
(22) Date of filing: 15.11.2000
(51) Int. Cl.: C07K 16/24, C12N 5/20, G01N 33/577, G01N 33/68, A61K 39/395

(54) **Antibody specific to interleukin 18 precursor**
Antikörper spezifisch für den Interleukin-18 Vorläufer
Anticorps contre le précurseur de l'interleukine-18

(30) Priority: 16.11.1999 JP 32486099
(43) Date of publication of application: 23.05.2001
(73) Proprietor: KABUSHIKI KAISHA HAYASHIBARA SEIBUTSU KAGAKU KENKYUJO, Okayama-shi, Okayama (JP)
(72) Inventor: Tohru, Kayano, Okayama-shi, Okayama (JP); Taniguchi, Mutsuko, Okayama-shi, Okayama (JP); Yamauchi, Hiroshi, Okayama-shi, Okayama (JP); Kurimoto, Masashi, Okayama-shi, Okayama (JP)
(74) Representative: Daniels, Jeffrey Nicholas

(56) References cited:
- EP-A- 0 821 005
- EP-A- 0 850 952
- WO-A-98/10072
- WO-A-99/09063
- M. TANIGUCHI ET AL.: "Characterization of anti-human interleukin-18 (IL-18)/interferon-gamma-inducing factor (IGIF) monoclonal antibodies and their application in the measurement of human IL-18 by ELISA." JOURNAL OF IMMUNOLOGICAL METHODS, vol. 206, no. 1-2, 1997, pages 107-113, XP004088140 Amsterdam, The Netherlands
- K. AKITA ET AL.: "Involvement of caspase-1 and caspase-3 in the production and procesing of mature human interleukin-18 in monocytic THP.1 cells." THE JOURNAL OF BIOLOGICAL CHEMISTRY, vol. 272, no. 42, 17 October 1997 (1997-10-17), pages 26595-26603, XP002159798 Baltimore, MD, USA
- H. OKAMURA ET AL.: "Cloning of a new cytokine that induces IFN-gamma production by T cells." NATURE, vol. 378, no. 6552, 2 November 1995 (1995-11-02), pages 88-91, XP002024313 London, GB
- S. USHIO ET AL.: "Cloning of the cDNA for human IFN-gamma-inducing factor, expression in Escherichia coli, and studies on the biologic activities of the protein." THE JOURNAL OF IMMUNOLOGY, vol. 156, no. 11, 1 June 1996 (1996-06-01), pages 4274-4279, XP002022861 Baltimore, MD, USA
- HIGGINS G. ET AL: 'Interleukin 1 beta propeptide is detected intracellularly and extracellularly when human monocytes are stimulated with LPS in vitro.' THE JOURNAL OF EXPERIMENTAL MEDICINE vol. 180, no. 2, 01 August 1994, pages 607 - 614, XP000982152

## Description

### Background of the Invention

### 1. Field of the Invention

This invention relates to a novel antibody, more particularly, an antibody specific to interleukin 18 precursor.

### 2. Description of the Prior Art

Interleukin 18 (hereinafter abbreviated as "IL-18") is a type of cytokine, which mediates signal transduction in immune system. As seen in Haruki Okamura et al., Nature, Vol.378, No.6552, pp.88-91 (1995), IL-18 was provisionally designated as "interferon γ inducing factor" immediately after its discovery; this designation was changed later into "IL-18" in accordance with the proposal in Shimpei Ushio et al., The Journal of Immunology, Vol.156, pp.4274-4279 (1996). IL-18 has a variety of biological activities, for example, inducing the production of interferon γ (hereinafter abbreviated as "IFN-γ") in immunocompetent cells, inducing the generation of killer cells, and enhancing the cytotoxicity of killer cells, as described in these publications.

The biosynthesis of IL-18 has been well investigated, as seen in Japanese Patent Kokai No.80,270/98 by the same applicant and Kenji Akita et al., The Journal of Biological Chemistry, Vol.272, No.42, pp.26595-26603 (1997). IL-18 is primarily produced in cells in the precursor form which has a molecular weight of about 24 kDa. When the precursor is exposed to the action of intracellular processing enzymes that remove N-terminal propeptide of the precursor, the precursor is converted into biologically active, mature form and then secreted from cells. The sequence listing includes examples of the amino acid sequences for human mature IL-18 and its precursor in SEQ ID NO:3 and 4, and those for murine counterparts in SEQ ID NOs:5 and 6. In SEQ ID NOs:1 and 2, the propeptide sequences of human and murine IL-18 precursors are separately shown.

Like as other cytokines, IL-18 activities are usually under strict regulation *in vivo* that prescribes the timings and sites to express the activities. The regulation would participate in keeping immune system and other physiological functions to work normally, and disordered expression of the activities would link with some diseases or disorders. For example, Masanori Kawashima et al. reported in Rheumatology in Europe, Journal for Education and Information in Rheumatology, vol.26, supplement No.2, p.77 (1997) that IL-18 levels in body fluids were elevated specifically in patients suffering from autoimmune diseases. This report suggests a correlation between the onset of autoimmune diseases and excessive expression of IL-18 activities in *vivo.* The process of converting IL-18 precursor into the mature form would be involved in the regulation of expressing IL-18 activities *in vivo*. Therefore, it is important for diagnoses of' IL-18-relating diseases to measure *in vivo* levels not only of mature IL-18 but also of IL-18 precursor in patients and compare the results with those in healthy specimens.

Japanese Patent Kokai Nos.217,798/96 and 231,598/96 by the same applicant disclose anti-mature-IL-18 antibodies including monoclonal antibodies and detection methods for polypeptides using the antibodies. The detection methods are useful in qualitative and quantitative analyses for mature TL-18. In contrast, there have been no established method to detect IL-18 precursor qualitatively or quantitatively; it is impossible to establish bioassays to detect the precursor, because it exhibit no activity, and there have never been disclosed any antibody specific to the precursor, feasible to detect the precursor immunologically. Non patent literature found in Nature; volume 373, no. 6552 pages 88-91 discloses claiming of a new cytokaine that induces IFN-γ production by T cells. IFN-γ-inducing factor (IGIF) augments natural killer activity in spleen cells. EP0821005 A2 teaches a method for converting a precursor of a polypeptide that induces IFN-γ production in immuno-competent cells, characterised in that it comprises a step of contacting an interleukin-1β converting enzyme with the precursor to convert it into an active polypeptide that induces IFN-γ product in immuno-competent cells.

In the Journal of Immumological Methods, volume 206, no. 1-2, on pages 107-113, characterisation of anti-human interleukin-18 monoclonal antibodies and their application in the measurement of human IL-18 by ELISA is taught. In order to develop a specific ELISA for the measurement of human IL-18, 13 anti-human IL-18 monoclonal antibodies were established and characterised. In the Journal of Experimental Medicine, volume 130, no. 2, on pages 607-614. The detection of Interleukin 1β propeptide intracellularly and extracellularly when human monocytes are immulated with LPS in vitro is taught.

The present inventors found through studying the detection methods for mature IL-18 that some anti-mature-IL-18 antibodies also exhibit an immunoreactivity against IL-18 precursor while the reaction level may be relatively low. This finding proved that detection methods using such antibodies can cause imprecise results, when employed on samples containing IL-18 together with the precursor. Methods of qualitative or quantitative analysis for IL-18 precursor were also demanded to correct the impreciseness.

### Summary of the Invention

In view of the foregoing, the first object of this invention is to provide an antibody which exhibits immunoreactivity against IL-18 precursor at a high specificity.

The second object of this invention is to provide processes to prepare the antibody.

The third object of this invention is to provide uses of the antibody.

The present inventors energetically studied to attain the above objections through immunizing non-human warm-blooded animals with IL-18 and its relating substances and comparing the antibodies produced therefrom in immunoreactivity. As a result, the inventors obtained antibodies specific to IL-18 precursor from body fluids of the animals which had been immunized with polypeptides comprising a part or whole of the propeptide amino acid sequence of the IL-18 precursor. Then, antibody-producing cells were colleoted from the animals pre-immunized similarly as above and fused with unlimitedly propagatable cells to generate hybridomas. The hybridomas thus obtained produced antibodies specific to IL-18 precursor. These antibodies were useful in various uses including the detection of IL-1B precursor. The present invention was established on the basis of these results.

In particular, the present invention attains the first object by providing an antibody specific to IL-18 precursor which is obtainable from a warm-blooded animal that has been immunized with a polypeptide comprising a part or the whole of the propeptide amino acid sequence of the IL-18 precursor.

The present invention attains the second object by providing a process to prepare an antibody which comprises the steps of immunizing a non-human warm-blooded animal with a polypeptide comprising a part or the whole of the propeptide amino acid sequence of the IL-18 precursor and collecting an antibody specific to IL-18 precursor from a body fluid of the immunized animal, and another process which comprises the steps of culturing *in vivo* except in human beings or *in vitro* an isolated cell capable of producing an antibody specific to IL-18 precursor and collecting the antibody from the resulting product of the *in vivo* except in human beings or in vitro culture.

The present invention attains the third object by providing a detection method and purification method for IL-18 precursor using an antibody specific to IL-18 precursor and pharmaceutical uses of the antibody.

### Brief Description of the Drawings

FIG. 1 illustrates the structure of the recombinant DNA "pRS-hproIL18", for expression of an antigen feasible to prepare the antibody of this invention.
FIG. 2 illustrates the structure of the recombinant DNA "pRS-hproIL18", for expression of another antigen feasible to prepare the antibody of this invention.
FIG. 3 shows the manner of selectively detecting IL-18 precursor by the detection method using the antibody of this invention "mAb-proHuIL18#75".

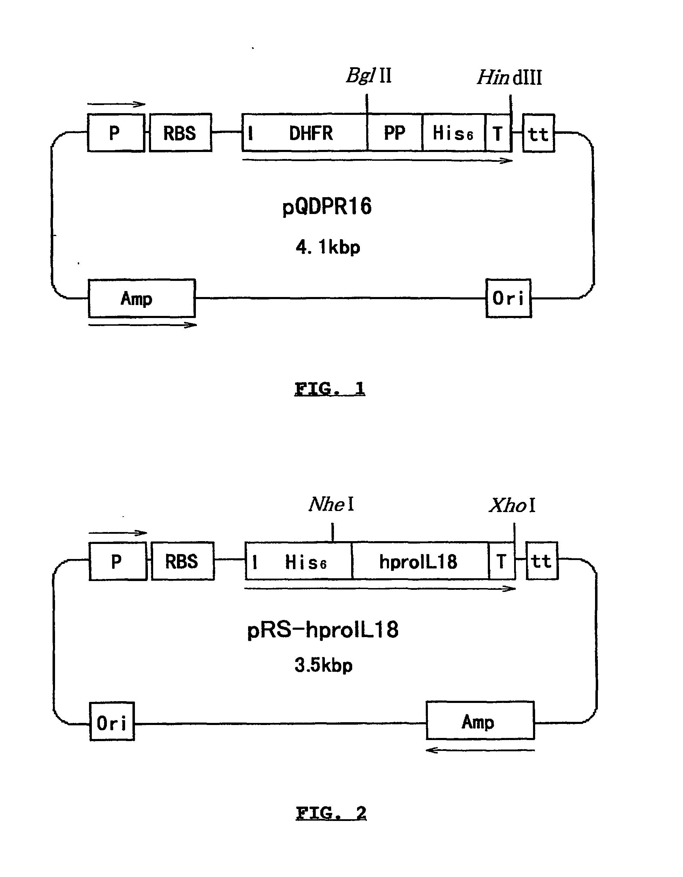

In FIGs. 1 and 2, "PP" indicates the coding region for the propeptide sequence of human IL-18 precursor; "hproIL18", the coding region for human IL-18 precursor; "His₆", the coding region for (His)₆ tag; "I", initiation codon; "T", termination codon; "DHFR", the coding region for mouse dihydrofolate reductase; "Ori", replication origin in *Escherichia coli*; "Amp", ampicillin resistant gene; "RBS", ribosome binding site; and "tt", transcription termination sequence.

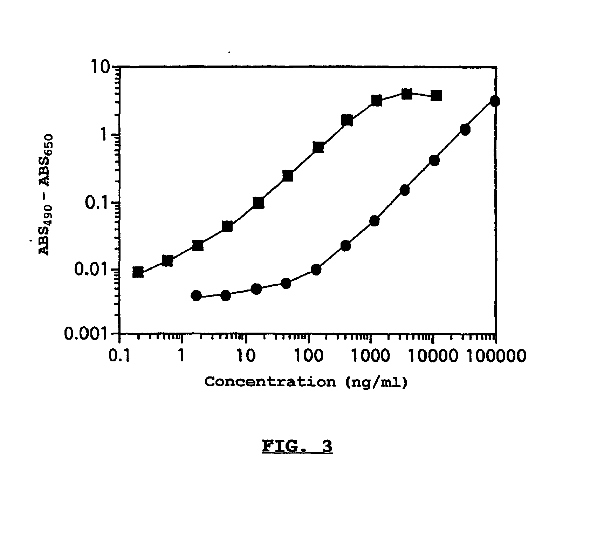

In FIG. 3, the axis of abscissas represents the concentration of human IL-18 precursor (solid squares) or human IL-18 (solid circles) added to well; and the axis of ordinates, the difference between absorbances at 490 nm and 650 nm of peroxidase-reacted mixture in well.

### Detailed Description of the Invention

This invention relates to an antibody specific to IL-18 precursor. The term "IL-18 precursor" as used in this invention refers to the precursor proteins produced intracellularly through the process of mature IL-18 biosynthesis and structural equivalents thereof regardless of their production processes. The term "mature IL-18" as used in this invention refers to the proteins, regardless of their origins, that comprise a sequence usually consisting of 157 amino acids and capable of inducing the production of IFN-γ in immunocompetent cells, inducing the generation of killer cells, and enhancing the cytotoxicity of killer cells. The sequence listing includes examples of the amino acid sequences for mature IL-18 in SEQ ID NOs:3 and 5, which are of a type of primate, human (Homo sapiens), and a type of rodent, mouse (Mus musculus), respectively (in SEQ ID NO:3, "Xaa" is threonine or isoleucine, and in SEQ ID NO:5, "Xaa" is methionine or threonine). The amino acid sequences shown in SEQ ID NOs:3 and 5 are homologous overall. These two sequences in which Xaa of SEQ ID NO:3 is threonine and Xaa of SEQ ID NO:5 is methionine show 101 identical amino acids when aligned in a usual manner for maximum matching. This means that the two sequences share 64.3% identity. The identity value indicates a feature of mature IL-18 in primary structure; mature IL-18 share usually at least 64.3%, preferably, at least 70%, and more preferably at least 80% identity with SEQ ID NO:3 or 5, calculated from the numbers of identical amino acids with SEQ ID NO:3 or 5 which are shown by conventional methods for maximum matching.

As described above, the term "IL-18 precursor" (hereinafter, occasionally designated "the precursor") refers to the precursor proteins of IL-18 (hereinafter, the term "IL-18" alone meaning identically with "mature IL-18"), which completely contain the amino acid sequence for IL-18 but exhibiting no biological activities as observed in IL-18. In primary structure, the precursor is characterized by the N-terminal extra sequence, or so-called propeptide sequence, added to the IL-18 sequence. The sequence listing includes examples of the propeptide sequence in SEQ ID NOs:1 and 2 that the precursor of human or mouse origin can contain and examples of the whole precursor sequence in SEQ ID NOs:4 and 6.

The antibody of this invention includes immunoglobulin molecules specific to the above defined IL-18 precursor in general, regardless of their origins, classes, or form (polyclonal or monoclonal antibody). The present antibody is obtainable from warm-blooded animals which have been immunized with a polypeptide comprising a part or the whole of the propeptide sequence of the precursor. The term "specific to IL-18 precursor" as used in this invention, for the characteristic of the present antibody, means that the immunoreactivity exhibited by the present antibody against IL-18 precursor is higher than that against any other substance. The term "immunoreaction" as used in this invention means the binding reaction between an antibody and a substance recognizable by the antibody, usually called antigen-antibody reaction, and the term "immunoreactivity" means the intensity of an immunoreaction. For example, when the levels of the immunoreactivity of the present antibody are compared between the cases against mature IL-18 and IL-18 precursor, the immunoreactivity against mature IL-18 is apparently lower than that against the precursor; at an intensity of, usually, at most 10%, preferably, at most 2%, and more preferably, at most 1% of that against the precursor. The levels of immunoreactivity can be compared quantitatively by conventional immunoassays, as described in detail in Examples later.

The present antibody can be easily prepared by the processes of this invention to prepare the present antibody, described below. From the processes, preferable one can be selected dependently on the objective type of the antibody.

Antigens for preparing the present antibody are not limited to those having specific structures as far as they comprise at least a part of the amino acid sequence for IL-18 precursor and can induce a desired warm-blooded animal to produce a desired antibody when administered to immunize the animal. Examples of the antigens include IL-18 precursor itself, fragments thereof containing at least a part of the propeptide sequence, and fusion proteins comprising at least one of the fragments with one or more of heterologous amino acid sequences. The wording "at least a part of the amino acid sequence for IL-18 precursor" means a partial sequence of IL-18 precursor or the propeptide sequence thereof which consists of a contiguous, usually, five or more, preferably, ten or more, and more preferably, 15 or more amino acids. The methods for preparing the antigen are not limited to specific types, and the antigen may be a recombinant or natural protein or synthesized peptide, for example. Recombinant proteins for the antigen can be obtained by conventional recombinant DNA techniques with isolated DNA molecules encoding IL-18 precursor, which are preparable from humans, mice, or others similarly as in Japanese Patent No.2,724,987 by the same applicant. SEQ ID NOs:7 and 13 show examples of the nucleotide sequence encoding IL-18 precursor of human or mouse origin. Japanese Patent Kokai No. 80, 270/98 by the same applicant discloses in detail the procedures to prepare human IL-18 precursor by recombinant DNA techniques. Fusion proteins for the antigen which comprise the propeptide sequence of IL-18 precursor and a heterologous sequence(s) can be obtained in accordance with Examples described later. IL-18 precursor proteins of animals other than humans can be obtained similarly as above with DNA molecules isolated from desired origins.

To prepare the antibody of this invention, in either form of a polyclonal or monoclonal antibody, first, warm-blooded animals can be immunized with an antigen by a conventional method. For example, an antigen can be injected alone or with an appropriate adjuvant using needles to the animals through intravenous, intracutaneous, subcutaneous, or intraperitoneal routes, and the animals can be housed for a prescribed period. The warm-blooded animals are not limited to specific species, sex, ages, or weights as far as they can produce the desired antibody. Examples of the warm-blooded animals are mammals including rodents such as mice, hamsters, rabbits, and guinea pigs and artiodactyls such as goats and sheep and birds including chickens and quails. Animals that are more suitable for immunization can be selected with respect to the origin of the antigen and the uses and objective form of the antibody, etc. The dosage of an antigen can be adjusted dependently on the species and weights of the target animals. When immunizing rodents, the total dosage is usually from 5 µg/head to 50 µg/head, which can be divided into two to 20 shots with each interval of about one to four weeks (in general, the first shot is called "primary immunization"; the shots after primary immunization, "additional immunizations"; and the final shot, "final immunization"). During and after immunization, the immunized animals can be examined for antibody productivity by conventional enzyme-immunoassay using the same antigen as in the immunization.

The present antibody in the form of a polyclonal antibody can be obtained by collecting sera (antisera) from the immunized animals through desired sites one to four weeks after immunization. The sites can be selected dependently on the species of immunized animals. The antisera can be further subjected, if necessary, to conventional methods to purify a desired class of immunoglobulin including IgG, IgA, and IgM, leading to producing the preparations of the polyclonal antibodies according to this invention at desired purification levels.

The present antibody can be also obtained from isolated cells capable of producing the present antibody. The term "isolated cells" as used in this invention refers to the cells existing apart from living bodies and capable of producing the present antibody, which include hybridomas, isolated spleen cells and lymphocytes, and transformant cells. This invention also provides such isolated cells. Any type of the isolated cells is useful to prepare the present antibody, and the hybridomas are particularly useful to prepare the monoclonal antibodies according to this invention.

To obtain the hybridomas, first, spleens can be extracted from the animals which have been immunized as above usually on day 3 to 5 after immunization and dispersed into spleen cells as antibody-producing cells. The spleen cells can be further immunized *in vitro,* if necessary. The spleen cells can be then fused with unlimitedly propagatable cells of a warm-blooded animal origin. The unlimitedly propagatable cells include, for example, cell lines established from mouse or rat myeloma, such as SP2/0-Ag14 (ATCC CRL-1631), P3/NSI/1-Ag4-1 (ATCC TIB-18), and P3X63Ag8 (ATCC TIB-9) and mutants thereof, from which more preferable ones can be selected with respect to compatibility to the above obtained spleen cells. To fuse the cells, desired methods can be selected from conventional ones such as those using cell-fusion accelerator including polyethylene glycol and Sendai virus or using electric pulse. The cell fusion products can be then subjected to cultivation with selection media such as HAT medium for selective propagation of fused cells, namely hybridomas. The propagated hybridomas can be examined with their culture supernatants for immunoreactivity against IL-18 precursor and IL-18. Hybridomas which exhibit desired immunoreactivity can be selected. The selected hybridomas can be cloned by conventional methods such as limiting dilution. The cloned hybridomas can be cultured *in vitro* or *in vivo*, and if necessary, the resulting cultures or body fluids can be purified by conventional methods to purify a desired class of immunoglobulin, leading to producing the preparations of the monoclonal antibodies according to this invention at desired purification levels.

Particular examples of methods conventionally used to purify antibodies include salting-out, dialysis, filtration, concentrating, centrifugation, separatory sedimentation, gel filtration chromatography, ion-exchange chromatography, affinity chromatography, high performance liquid chromatography, gel electrophoresis, and isoelectric focusing, which can be arbitrarily used for this invention, if necessary, in combination with one another. The purified preparations can be concentrated or dehydrated into the desired form of a liquid, solid, or others dependently on their uses.

The present antibody can be prepared by other methods. For example, a variety of established methods using recombinant DNA techniques can be arbitrarily applied to the preparation of the present antibody, particularly, those in the form of a monoclonal antibody. In brief, first, isolated cells capable of producing the present antibody are prepared as described above, by isolating spleen cells from pre-immunized, warm-blooded animals or establishing hybridomas or by immunizing in vitro with an antigen lymphocytes collected from warm-blooded animals including humans. Then, RNA is collected from the isolated antibody-producing cells, and DNA molecules which encode portions comprising the complementarity-determining regions of a molecule of the present antibody can be cloned from the RNA used as a template, for example, by RT-PCR method described in S. Tarran Jhones et al., Bio/technology, Vol.9, pp.88-89 (1991) or screening the expression library described in S. Paul, Methods in Molecular Biology, Vol.51, pp.355-394 (1995; Humana press Inc., Totowa, New Jersey, USA), which is usually called phage display library. Ligation of the cloned DNA molecules with a DNA molecule encoding a constant region of a desired immunoglobulin molecule can produce recombinant DNAs which encode polypeptides for the present antibody. Techniques for protein engineering can be conducted with the obtained recombinant DNA to alter the present antibody in primary structure as far as not substantially losing their specificity to IL-18 precursor. The present antibody by recombinant DNA techniques can be obtained by the steps of bringing the obtained recombinant DNAs into expression in desired expression systems and purifying the expressed antibodies. These recombinant techniques, combined with one another if desired, can produce not only antibodies equivalent in structure and function to those produced by warm-blooded animals other than humans but also antibodies comprising human constant regions, namely, "humanized antibodies" and "chimeric antibodies", and antibodies substantially equivalent to those produced by human lymphocytes, namely, "human antibodies". Tristan J. Vaughan et al. illustrate humanized, chimeric, and human antibodies and preparation methods therefor in Nature Biotechnology, Vol.16, pp.535-539 (1998). These methods can be arbitrarily applied to the preparation of the present antibody as above. The present antibody in the form of a chimeric, humanized, or human antibody is particularly useful in pharmaceutical uses for humans.

The present antibody, obtainable as above, can be used in a wide variety of fields where the detection of IL-18 precursor is demanded. Because IL-18 precursor usually exhibits no biological activity as observed in IL-18, no bioassay to detect the precursor is available. By using the present antibody, specific to IL-18 precursor, the detection of the precursor becomes possible. This invention also provides the detection method for IL-18 precursor, which comprises the steps of contacting the antibody with samples and detecting the precursor on the basis of the resulting immunoreaction or its intensity. The present detection method can be performed with the present antibody in accordance with, for example, conventional immunoassays which use a label to detect the immunoreactions or measure the intensities thereof. The conventional immunoassays include those using a radioactive substance, enzyme, or fluorescent substance as a label, which are called in general radioimmunoassay, enzyme-immunoassay, and fluoroimmunoassay. Enzyme-immunoassay may also be called "ELISA (enzyme-linked immunosorbent assay)". Other examples are those using different labels, for example, metals such as colloidal gold and chelates containing metal ion such as europium ion and samarium ion. In these immunoassays, the present antibody can be labeled before use directly with a desired one or more of the labels as shown above. Alternatively, the present antibody can be labeled indirectly. For example, the present antibody is to be labeled indirectly when used in the form bound with biotin, a type of vitamin, and contacted with a label-bound avidin, a substance having affinity to biotin. Because the present antibody is specific to IL-18 precursor, such immunoassays using the present antibody can qualitatively or quantitatively detect IL-18 precursor, contained even at low levels in samples, at a desirable preciseness without wasting time by detecting or measuring the immunoreactions on the basis of the label used.

Conventional immunoassays may be performed with different two or more types of antibodies, as in so-called sandwich immunoassay. The present detection method also can be performed similarly, for example, with the monoclonal antibodies according to this invention produced by different hybridomas and/or with the polyclonal antibodies according to this invention in different preparations. Further, as described in Example 2 later, IL-18 precursor may be detected at a desirable sensitivity and selectivity by using an anti-IL-18 antibody in combination with a type of the present antibody, while the effectiveness varies dependently on the immunological properties of the antibodies used. With regard to these, the present detection method includes those which detect IL-18 precursor as far as using at least one type of the present antibody, independently of the types of other antibodies used in combination.

Available anti-IL-18 antibodies may exhibit an immunoreactivity against IL-18 precursor, whereas the reactivity may be relatively low. When immunoassays are performed with such antibodies on specimens which contain IL-18 and the precursor, the measured values would include impreciseness. The detection method of this invention makes it possible to correct the imprecise results or values caused by such immunoassays. Therefore, the detection method of this invention is effective in analyses not only for IL-18 precursor but also for IL-18.

The detection method of this invention is useful, for example: in diagnoses of diseases relating to mature IL-18 or IL-18 precursor, which may be associated with the enhancement or attenuation of IL-18 production *in vivo* or with disorders in the conversion of IL-18 precursor to the mature form; in analyses for mechanisms to progress such diseases; and in quality control of final and intermediate products in industrial or experimental productions of IL-18 through synthesizing IL-18 precursor. Examples of specimens analyzable by the present detection method include biological samples, in particular, those collected from living bodies such as bloods, sera, lymphs, bone marrow fluids, salivas, sweats, stools, cells, tissues, organs, and lysates of cells, tissues, and organs and those from *in vitro* cultures such as cultured cells and cell lines, culture supernatants thereof, and cell lysates therefrom.

The present antibody exhibits a particular efficacy to conventionally detect IL-18 precursor when incorporated in an immunoassay kit with other reagents to effect the immunoassay for IL-18 precursor, for example, another antibody and buffers, if necessary, in combination with reagents for labeling, reagents to detect the label, plates, and IL-18 precursor for standard, and if further necessary, with anti-IL-18 antibodies and IL-18. This invention also provides the kit. General procedures for immunoassay including methods for antibody preparation are described, for example, in Practical Immunoassay, edited by Wilfrid R. Butt, published by Marcel Dekker, Inc., New York, USA (1984), and in pp.37-69 of this text, enzyme-immunoassay is particularly described. These general procedures can be arbitrarily applied to the present detection method.

The present antibody is also useful in immunoaffinity chromatography to purify IL-18 precursor. The purification method of this invention comprises the steps of contacting the present antibody with a sample containing IL-18 precursor and impurities to adsorb the precursor selectively on the antibody and desorbing the precursor from the antibody, both which are usually performed in aqueous media. For this method, the present antibody is usually used after immobilized on a water-insoluble carrier. To the antibody-immobilized carriers applying mixtures containing IL-18 precursor such as cultured transformants and partial purified preparations thereof brings the precursor to being adsorbed to the immobilized antibody in a substantially exclusive manner. The adsorbed substances can be easily desorbed by altering pH around the antibody. For example, in the case of using antibody belonging to IgG, the adsorbed substances can be desorbed from the antibody by controlling acidic conditions, usually pH 2-3; and in the case of antibody belonging to IgM, by controlling basic conditions, usually pH 10-11. By this method, IL-18 precursor can be relatively easily purified at relatively high purity.

Whereas IL-18 exhibits various activities *in vivo*, for example, involved in regulating immune system, IL-18 may affect body health when IL-18 activities is over or under normal ranges *in vivo.* This is well evidenced by the finding that suggests a correlation between the onset of autoimmune diseases and elevated IL-18 levels in *vivo.* It is also likely that body health is affected by the accumulation of IL-18 precursor in body fluids or cells, which may be caused by a disorder in the conversion of IL-18 precursor to the mature form. For example, accumulated IL-18 precursor may inhibit IL-18 activities, possibly resulting in disorders or diseases. The present antibody, specific to IL-18 precursor, can eliminate or detoxify the precursor accumulated *in vivo.* Therefore, treatments with the present antibody can remedy, alleviate, or prevent disorders or diseases caused by IL-18 precursor accumulated *in vivo.* In addition, IL-18 precursor may suppress the therapeutic effects of IL-18 inhibitors or neutralizers for IL-18-relating diseases when the precursor excessively exists in the patients at sites where the precursor encounters the administered inhibitors or neutralizers. Therefore, the present antibody can optimize the therapeutic effects of the inhibitors and neutralizers in such cases by blocking the suppressive actions of the precursor. With regard to these, the present antibody can be beneficially used as an effective or adjuvant ingredient for pharmaceutical compositions in general to remedy, alleviate, or prevent the diseases of humans and other animals against which the present antibody directly or indirectly effects, such as diseases relating to IL-18 precursor or IL-18. The above described, humanized, chimeric, and human antibodies according to this invention are particularly efficacious in such pharmaceutical uses for humans because the antibodies are less antigenic to humans than antibodies of any other origin. In pharmaceutical uses for animals other than humans, it is preferable to employ the present antibody of an origin corresponding to the species of animals to be treated.

This invention also provides a pharmaceutical composition comprising the present antibody, having the usefulness as above. Particular examples of diseases which can be treated with the present pharmaceutical composition include: viral infections such as hepatitis, herpes, condyloma acuminatum, and acquired immunodeficiency syndrome; bacterial infections such as candidiasis and malaria; solid malignant tumors such as epinephroma, mycosis fungoides, and chronic granulomatous disease; malignant tumors of hemocytes such as adult T-cell leukemia, acute lymphocytic leukemia, acute myelocytic leukemia, chronic myelocytic leukemia, non-T-lymphocytic leukemia, non-Hodgkin's lymphoma, multiple myeloma, and malignant lymphoma; sarcomas, sarcomatosis, sarcoids, or sarcoidosis such as leiomyosarcoma, fibrosarcoma, ophthalmic sarcoidosis, and pulmonary sarcoidosis; autoimmune diseases, allergic diseases or immune diseases such as rejection reactions against grafts, chronic graft-versus-host disease, pernicious anemia, atrophic gastritis, insulin resistant diabetes, Wegener's granulomatosis, discoid lupus erythematosus, hemophagocytic syndrome, idiopathic ulcerative colitis, cold agglutinin disease, Goodpasture's syndrome, Crohn's disease, sympathetic ophthalmia, hyperthyroidism, juvenile diabetes, Sjögren's syndrome, autoimmune hepatitis, autoimmune hemolytic anemia, myasthenia gravis, progressive systemic sclerosis, systemic lupus erythematosus, multiple cold hemoglobinuria, polymyositis, multiple nodal arteritis, idiopathic Addison's disease, idiopathic thrombocytopenic purpura, Basedow's disease, leukopenia, Behcet's disease, climacterium praecox, rheumatoid arthritis, adult Still's disease, Still's disease, rheumatopyra, chronic thyroiditis, Hodgkin's disease, DiGeorge's syndrome, acute graft-versus-host disease, HIV infection, asthma, atopic dermatitis, contact dermatitis, allergic rhinitis, pollinosis, and apitoxin allergy; hepatopathies such as viral hepatitis, alcoholic hepatitis, toxic hepatitis, primary biliary cirrhosis, fulminant hepatitis, viral cirrhosis, alcoholic cirrhosis, toxic cirrhosis, biliary cirrhosis, cholestatic hepatopathy, hepatocyte tumor, acute hepatitis, fatty liver, and hepatoma; diseases in gallbladder or biliary tract such as cholangitis, cholecystitis, primary sclerosing cholangitis, carcinoma of gallbladder, and cholangiocarcinoma; pancreatopathies such as acute pancreatitis, chronic pancreatitis, pancreatic failure, and pancreatic cysts; diseases in kidney or glomerulus such as acute nephritis, chronic renal failure, renal cancer, renal ischemia, nephrolithiasis, glomerulonephritis, glomerulitis, and glomerulosclerosis; diseases relating to circulating organs such as ischemia, congestive cardiomyopathy, cerebral ischemia, basilar artery migraine, abnormal vascularnet at brain base, cerebral apoplexy, aneurysm in basilar artery, arteriosclerosis, endangiitis, diabetes, mesenteric vascular occlusion, and superior mesenteric artery syndrome; and diseases relating to nervous system such as Parkinson's disease, myelatrophy, amyotrophic lateral sclerosis, Alzheimer's disease, dementia, cerebrovascular dementia, AIDS dementia, and encephalomyelitis. In addition, these diseases can also be diagnosed by the above described detection method of this invention.

The present pharmaceutical composition is provided in either form comprising the present antibody alone or in combination with one or more of physiologically acceptable, for example, carriers, excipients, diluents, solvents, adjuvants, and stabilizers, and if necessary, further with one or more of biologically active substances. Particular examples of the stabilizers include proteins such as serum albumen and gelatins, saccharides such as glucose, sucrose, lactose, maltose, trehalose, sorbitol, maltitol, mannitol, and lactitol, and buffers involving citrate or phosphate. Particular examples of the biological active substances include FK506, glucocorticoid, cyclophosphamide, nitrogen mustard, triethylenethiophosphoramide, busulfan, pheniramine mustard, chlorambucil, azathioprine, 6-mercaptopurine, 6-thioguanine, 6-azaguanine, 8-azaguanine, 5-fluorouracil, cytarabine, methotrexate, aminopterin, mitomycin C, daunorubicin hydrochloride, actinomycin D, chromomycin A3, bleomycin hydrochloride, doxorubicin hydrochloride, cyclosporin A, L-asparaginase, vincristine, vinblastine, hydroxyurea, procarbazine hydrochloride, adrenocortical hormone, colloidal gold, interleukin 2, interleukin 12, interleukin 18, receptor antagonists and neutralizers for cytokines, including antibodies against interleukin 1 receptor proteins, interleukin 2 receptor proteins, interleukin 5 receptor proteins, interleukin 6 receptor proteins, interleukin 8 receptor proteins and interleukin 12 receptor proteins, respectively, and antagonists for TNF-α receptors, TNF-β receptors, interleukin 1 receptors, interleukin 5 receptors and interleukin 8 receptors. The present pharmaceutical composition contains the present antibody at a content of, usually, 0.00001%(w/w) to 100%(w/w), preferably, 0.0001%(w/w) to 20%(w/w) on the dry solid basis dependently on the species and ages of patients and the types of diseases to be treated.

The pharmaceutical composition of this invention includes pharmaceutics having a minimal unit for medication, which are in physically united formula suitable for one shot of administration and containing the present antibody at multiples (up to fourfold) or divisor (not less than 1/40) of a prescribed dose. Particular examples of the formulas of the present pharmaceutical composition include injections, liquids, powders, granules, syrups, tablets, capsules, and external agents, etc. The doses of the present pharmaceutical composition can be adjusted dependently on the species and ages of patients and the types of diseases to be treated. For example, to treat adult humans, the present pharmaceutical composition can be administered at a dose, per shot per body on the dry solid basis of the present antibody, in the range of, usually, about 0.1 µg to about 1 g, preferably, about 1 pg to about 100 mg, with a frequency of 1 to 4 shots a day or 1 to 30 shots a week for a period over one day to one year, while the symptoms can be monitored. The administration routes are not specifically limited as far as leading to desired remedial, alleviative, or preventive effects, and the routes can be selected from, for example, oral routes and paraoral routes such as intradermal, subcutaneous, intramuscular, and intravenous routes dependently on the diseases to be treated or the formulas of the composition to be administered.

The present antibody may exhibit a cross reactivity, for example, against substances similar but not identical to IL-18 precursor in whole structure or sharing a partial structure with the precursor. The antibody of this invention exhibiting such cross reactivity is feasible in screening for IL-18-precursor-relating substances. In the screening, for example, biological samples such as cells, culture supernatants of cells, cell lysates, bloods, sera, tissues, tissue lysates, etc., and samples of peptide having at random sequences consisting of several to several tens of amino acids can be examined for immunoreactivity with the present antibody. Therefore, the present antibody can be beneficially used as a reagent to screen for the substances structurally or immunologically relating to IL-18 precursor. The substances obtainable through the screening, including small molecules such as peptides, may participate in the expression of IL-18 activities by acting as its agonist, antagonist, inhibitor, or neutralizer, so that the substances could be used as an IL-18-regulator or agent for diseases relating to IL-18. In addition, analyzing and comparing the substances in structure and function can contribute to molecular designing for further different IL-18-regulators.

The followings are particular examples of this invention. This invention is not limited to these examples, because the examples can be variously modified with regard to the technical levels in this field.

### Example 1

### Antibody specific to IL-18 precursor

### Example 1-1

### Preparation of antigen

A fusion protein comprising a part of the propeptide sequence of human IL-18 precursor shown in SEQ ID NO:1 linked with mouse dihydrofolate reductase (hereinafter abbreviated as "DHFR") and (His)₆ tag was prepared by recombinant DNA techniques as follows for an antigen to prepare an antibody specific to human IL-18 precursor.

### Example 1-1(a)

### Preparation of DNA coding for antigen

A DNA fragment encoding the propeptide sequence other than the N-terminal methionine was amplified by a conventional PCR, in which human IL-18 cDNA comprising SEQ ID NO:7 prepared as described in Japanese Patent No.2,724,987 by the same applicant was used as a template and oligonucleotides shown in SEQ ID NOs:8 and 9 was used as sense and antisense primers, respectively. The sense primer was designed to encode N-terminus of SEQ ID NO:1 excepting the first methionine and contain the recognition site by *Bgl*II; the antisense primer, C-terminus of SEQ ID NO:1 followed by (His)₆ tag and the recognition site by *Hind*III. Amplified DNA fragment was cloned in a conventional cloning vector and sequenced. The DNA fragment had the desired structure.

The DNA fragment was excised from the cloning vector by digestion with *Bgl*II and *Hin*dIII and inserted into *Bgl*II-*Hin*dIII-digested "pQE-16", an expression vector for *Escherichia coli* host containing mouse DHFR gene which is located under regulation of a promotor and followed by the recognition sites by *Bgl*II and *Hin*dIII, purchased from Kabushiki Kaisha QIAGEN, Tokyo, Japan. As shown in FIG. 1, in the recombinant DNA thus obtained, there existed downstream of the promotor the amino acid sequences for mouse DHFR, a part of the propeptide of human IL-18 precursor, and (His)₆ tag. Thus obtained recombinant DNA was named "pQDPR16".

### Example 1-1(b)

### Expression and purification of antigen

The recombinant DNA pQDPR16, obtained in Example 1-1(a), was introduced into *Escherichia coli* strain "JM109" by a conventional method. The transformant was pre-cultured in L broth, and 2.4 liters of 2 × YT medium prepared in 500-ml-Erlenmeyer flasks at 150 ml/flask was seeded with the pre-culture at 1% by volume and incubated at 37°C with a rotary shaker to culture the transformant, while the medium was monitored for the absorbance at 600 nm. When the absorbance reached 0.9, IPTG was added to the medium to give the final concentration of 1 mM and the incubation was further continued overnight. From the resulting culture, the cells were collected. After the cells (12.1 g by wet weight) were suspended in 480 ml of a buffer (pH 8.0) containing 8 M urea, from the suspension the expression product by the transformant was purified through Ni-NTA agarose gel column (12 ml by gel volume, QIAGEN) in accordance with manufacturer's instructions. The column-purified preparation was then dialyzed at 4°C overnight against 10 mM Tris-HCl buffer (pH 7.2) containing 0.05%(w/v) detergent "TWEEN 20", 5 mM 2-mercaptoethanol, 0.5 M NaCl, and 1.7 M urea. The dialyzed solution was centrifuged, and the supernatant was concentrated by ultrafiltration to give a protein concentration of about 1 mg/ml. The concentrate was centrifuged, and the supernatant was collected. The collected preparation (16 ml) contained 14.6 mg of protein, which exhibited an about 33 kDa band homogeneous on sodium dodecyl sulfate-polyacrylamide gel electrophoresis (hereinafter abbreviated as "SDS-PAGE") in the presence of dithiothreitol (hereinafter abbreviated as "DTT").

For control, another manipulation was conducted as above with the expression vector pQE-16 in place of the recombinant DNA pQDPR16. This manipulation produced an about 28 kDa protein homogeneous on SDS-PAGE in the presence of DTT. This result supports that the above obtained concentrate is a purified preparation of the fusion protein, comprising a part of SEQ ID NO:1 for the propeptide sequence, mouse DHFR, and (His)₆ tag. Thus an antigen was purified.

### Example 1-2

### Preparation of DNA coding for antigen

A fusion protein comprising the whole amino acid sequence for human IL-18 precursor, shown in SEQ ID NO:4, and (His)₆ tag was prepared by recombinant DNA techniques as follows for another antigen.

### Example 1-2(a)

### Preparation of DNA coding for antigen

To amplify a DNA fragment that contains a coding sequence for human IL-18 precursor shown in SEQ ID NO:4 and the recognition sites by *Nhe*I and *Xho*I added respectively to the 5'-and 3'-termini, the following PCR was conducted. A PCR mixture in the volume of 100 µl containing 1 ng of cDNA comprising the nucleotide sequence of SEQ ID NO:7, prepared as described in Japanese Patent No.2,724,987 by the same applicant, 10 ng of the oligonucleotide shown in SEQ ID NO:10 as a sense primer, 100 ng of the oligonucleotide shown in SEQ ID NO:11 as an antisense primer, and other components as in conventional PCR at usual prescriptions was subjected to five cycles of incubation at 94°C for 30 sec, at 53°C for 30 sec, and at 72°C for 30 sec. To the resulting PCR mixture, 100 ng of the oligonucleotide of SEQ ID NO:12 was added, and the mixture was subjected to three cycles of incubations at 94°C for 30 sec, at 55°C for 30 sec, and at 72°C for 30 sec and then 32 cycles of incubations at 94°C for 30 sec, at 60°C for 30 sec, and at 72°C for 30 sec. Amplified DNA fragment was cloned in a conventional cloning vector and sequenced. The DNA fragment had the desired structure.

The DNA fragment was excised from the cloning vector by digestion with *Nhe*I and *Xho*I and then inserted in a usual manner into *Nhe*I-*Xho*I-digested "pRSETB", an expression vector for *Escherichia coli* host which comprises a promotor-regulated coding sequence containing an initiation codon and a sequence for (His)₆ tag followed by the recognition sites by *Nhe*I and *Xho*I, commercialized by Invitrogen Corporation, San Diego, USA. As shown in FIG. 2, in the obtained recombinant DNA, there existed under regulation of the promotor a coding sequence for an amino acid sequence consisting of human IL-18 precursor and another sequence containing (His)₆ tag that is linked at the N-terminal of the precursor. The recombinant DNA was named "pRS-hproIL18".

### Example 1-2(b)

### Expression and purification of antigen

The recombinant DNA pRS-hproIL18, obtained in Example 1-2(a), was introduced into *Escherichia coli* strain "BL21(DE3)pLysS" in competent cells, purchased from TOYOBO Co., Osaka, Japan. The transformant was pre-cultured in L broth, and one liter of L broth prepared in 500-ml-Erlenmeyer flasks at 150 ml/flask was seeded with the pre-culture at 1% by volume and incubated at 37°C with a rotary shaker to culture the transformant, while the medium was monitored for absorbance at 600 nm. When the absorbance reached 0.5, IPTG was added to the medium to give a final concentration of 0.4 mM and the incubation was continued further five hours. From the resulting culture, the cells were collected. After the cells (12.1 g by wet weight) were suspended in 40 ml of 0.01 M Tris buffer (pH 8.0) containing 0.5 M urea and 0.1 M NaH₂PO₄ and sonicated, the suspension was centrifuged to precipitate the inclusion body fraction. The inclusion body fraction was washed by once repeating the treatment of suspension, sonication, and centrifugation. The washed inclusion body fraction was solubilized with 10 ml of a buffer (pH 8.0) containing 8 M urea, and from the resulting solution, the expression product by the transformant was purified through Ni-NTA agarose gel column (5 ml by gel volume, QIAGEN) in accordance with the manufacturer's instructions. The column-purified preparation was dialyzed at 4°C overnight against 10 m Tris buffer (pH 7.2) containing 5 mM EDTA and 0.1 M NaH₂PO₄. The dialyzed solution was centrifuged to collect the supernatant, and the supernatant was concentrated by ultrafiltration to a protein concentration of about 1 mg/ml, about 4 ml in volume. The concentrate exhibited an about 30 kDa band homogenous on SDS-PAGE in the presence of DTT.

For control, another manipulation was conducted as above with the expression vector pRSETB in place of the recombinant DNA pRS-hproIL18. This manipulation did not produce the protein remarkably visible on SDS-PAGE in the presence of DTT as observed in the case with pRS-hproIL18. This supports that the above obtained concentrate is a purified preparation of the fusion protein, comprising human IL-18 precursor. Thus another antigen was purified.

### Example 1-3(a)

### Immunization of mice and preparation of antibody-producing cells

Eight-week-old female BALB/c mice were immunized as follows. For primary immunization, the antigen prepared in Example 1-1 was injected with needles in a usual manner in combination with complete Freund adjuvant to mice through intraperitoneal routes at 20 µg/head. Every two weeks thereafter, the antigen was injected twice to the mice for additional immunizations with incomplete Freund adjuvant through the same routes and at the same dose as the primary immunization. Bloods were collected from the mice through tail veins in a usual manner, and the sera prepared therefrom were examined for immunoreactivity against the antigen. Elevation of the desired antibody titer was observed in mice. These results mean that the sera are antisera containing polyclonal antibodies specific to human IL-18 precursor and that the polyclonal antibodies of this invention are obtainable in accordance with this Example.

The mice received additional immunizations further twice similarly as above. Two weeks thereafter, the other antigen, prepared in Example 1-2, was injected once to the mice for final immunization at 10 µg/head without adjuvant. On day 3 after the final immunization, spleens were extracted from the mice and dispersed into spleen cells as antibody producing cells.

### Example 1-3(b)

### Establishment of hybridomas

The spleen cells obtained from one mouse were suspended in serum-free RPMI1640 medium (pH 7.2, hereinafter designated "the serum-free medium"), preheated to 37°C, together with the murine bone marrow cells SP2/0-Agl4 (ATCC CRL1581) to give the respective cell densities of 1×10⁸ cells/ml and 3×10⁷ cells/ml, and the suspension was mixed well. The suspension was centrifuged, and the supernatant was removed. The cells in pellet were allowed to fuse by receiving drop by drop 1 ml of the serum-free medium containing 50%(w/v) polyethylene glycol having the mean molecular weight of 1,500 daltons over one minute and then being incubated at 37°C for one minute. The cell fusing reaction was terminated by adding the serum-free medium to the mixture to give the final volume of 50 ml. The cell fusion product was centrifuged to separate completely from the supernatant, and the pellet was suspended in HAT medium to give a cell density of about 2×10⁵ cells/ml. The cell suspension was divided in 96-well microplates at 150 µl/well and incubated in a 5%(v/v) CO₂ incubator at 37°C for about 10 days to let hybridomas selectively propagate.

The culture supernatants of the wells were examined for immunoreactivity against human IL-18 precursor and human IL-18 by conventional immunoassay as follows. Human IL-18 precursor, consisting of the amino acid sequence shown in SEQ ID NO:4, was prepared as described in Japanese Patent Kokai No.80,270/98 by the same applicant and immobilized in a usual manner in the wells of plastic plates. Human IL-18, comprising the amino acid sequence shown in SEQ ID NO:3, was prepared as described in Japanese Patent No.2,724,987 by the same applicant and immobilized as above in the wells of other plastic plates. Each well of the plates was given with a portion of any culture supernatant of the hybridomas and then washed. The wells were further given with goat anti-mouse-IgG antibody labeled with horseradish peroxidase and then washed. The wells were subjected to peroxidase reaction using o-phenylenediamine and peroxide as substrates. On the basis of the color development after the reaction, immunoreaction was judged whether occurred or not. Fifteen samples exhibited an immunoreactivity against human IL-18 precursor but not apparently against human IL-18. From the wells of the hybridoma culture that contained these samples, hybridomas were respectively collected and subjected to limiting dilution, leading to establishment of 15 independent clones of hybridomas. The clones were cultured and analyzed in a usual manner. One clone produced the IgG_{2b} class of monoclonal antibody; two clones, IgG₃; and the remaining 12 clones, IgM.

### Example 1-3(c)

### Preparation of monoclonal antibodies

The hybridomas obtained as 15 independent clones in Example 1-3(b) were manipulated to prepare monoclonal antibodies as follows. Each hybridoma was suspended in RPMI1640 medium (pH 7.2) supplemented with 5%(v/v) fetal calf serum to give a cell density of 1×10⁵ cells/ml and cultured at 37°C in a 5%(v/v) CO₂ incubator to a prescribed cell numbers. Eleven-week-old BALB/c mice received pristane at 0.5 ml/head through intraperitoneal routes. Each hybridoma was collected from the culture and injected to the pristane-injected mice at 1×10⁷ cells/head through intraperitoneal routes. The mice were housed for about one week in a usual manner.

Monoclonal antibodies from the mice that received hybridomas producing the IgG_{2b} or IgG₃ class of monoclonal antibodies were purified as follows. A column packed with the protein-A-bound gel "Protein A-Sepharose CL-4B" (Pharmacia LKB Biotechnology AB, Uppsala, Sweden) was equilibrated with 1.5 M glycine-NaOH buffer (pH 8.9) containing 3 M NaCl (hereinafter, designated "the equilibration buffer"). Ascites was collected from each housed mouse, purified by centrifugation in a usual manner, twofold diluted with the equilibration buffer, and applied to the equilibrated column. The column was washed with an adequate amount of the equilibration buffer, and then an appropriate amount of 0.1 M glycine-HCl buffer (pH 3.0) was run to elute the antibody adsorbed in the column. The eluate was recovered and dialyzed against PBS (phosphate-buffered saline) at 4°C overnight. Thereafter the dialyzed solution was recovered. These manipulations were conducted with every hybridoma, resulting in purified preparations of three independent monoclonal antibodies: one belongs to the class IgG_{2b}; and remaining two, IgG₃.

From mice that received hybridomas producing the IgM class of monoclonal antibodies, ascites was collected, purified by centrifugation in a usual manner, and salted-out with 50%-saturated ammonium sulfate, and then the resulting precipitate was collected. The precipitate fraction was dialyzed at 4°C overnight against 50 mM phosphate-potassium buffer (pH 6.8) containing 0.15 M NaCl. The dialyzed solution was applied to a hydroxyapatite column equilibrated with the same buffer as above, and the column was washed with an adequate amount of the same buffer. Through the column, phosphate-potassium buffer (pH 6.8) containing 0.15 M NaCl was run at the buffer concentration increasing from 50 mM to 300 mM in a linear gradient manner, and fractions eluted with the buffer at over 200 mM were collected and pooled. These manipulations were conducted with every hybridoma, resulting in purified preparations of 12 independent monoclonal antibodies which belong to the class IgM.

The monoclonal antibodies obtained in this Example were subjected to enzyme-immunoassay as described in Example 1-3(b). These monoclonal antibodies exhibited an immunoreactivity against human IL-18 precursor but not apparently against human IL-18. Thus 15 types of the present antibody were obtained. One of the monoclonal antibody belonging to the class IgG_{2b} whose immunoreactivity against IL-18 precursor was particularly high, in this assay, was named "mAb-proHuIL18#75".

### Example 2

### Enzyme-immunoassay

Enzyme-immunoassay was conducted with two types of antibodies as follows. For the first antibody, the monoclonal antibody mAb-proHuIL18#75 was prepared in accordance with Example 1-3(c). Anti-human-IL-18 monoclonal antibodies against were prepared as described in Japanese Patent Kokai No.231,598/96 by the same applicant. The anti-human-IL-18 monoclonal antibodies were subjected to western blotting in a usual manner. One sample, named "mAb-HuIL18#25-2G", exhibited an immunoreactivity against human IL-18 as well as against human IL-18 precursor at an intensity comparable to that against human IL-18. For the second antibody, the monoclonal antibody mAb-HuIL18#25-2G was labeled with horseradish peroxidase in a usual manner. For test samples, human IL-18 precursor was prepared similarly as in Japanese Patent Kokai No.80,270/98 by the same applicant, and human IL-18 precursor, in accordance with Japanese Patent No.2,724,987 by the same applicant.

The first antibody was prepared in a 20 µg/ml solution with PBS and poured in microplates at 100 µl/well. The microplates were allowed to stand at ambient temperature for three hours so that the antibody was immobilized in wells. The wells were washed with PBS, added with PBS containing 0.1%(w/v) bovine serum albumin at 200 µl/well, and allowed to stand at 4°C for 16 hours. The wells were washed with PBS, and to each well the following materials were added: 50 µl of a solution containing human IL-18 precursor or human IL-18 prepared at any concentration shown in FIG. 3 and 50 µl of the assay buffer (PBS containing 1%(w/v) BSA, 5%(w/v) fetal calf serum, and 1 M NaCl). The microplates were gently rotated at ambient temperature for one hour. The wells were washed thrice with the washing buffer (PBS containing 0.05%(w/v) detergent "TWEEN 20"), and the second antibody, prepared and labeled above, was added to the wells at 100 µl/well. The microplates were rotated at ambient temperature for 1.5 hour. The wells were washed thrice with the washing buffer and then subjected to peroxidase reaction in a usual manner using o-phenylenediamine and peroxide as substrates. After the reaction, the reaction mixtures were examined for absorbances at 490 nm and 650 nm, and the differences between the former and latter absorbances were calculated to indicate the intensities of immunoreaction occurred in the wells.

FIG. 3 summarizes the results in the cases with human IL-18 precursor (solid squares) and those with human IL-18 (solid circles). Whereas not shown, the differences between the absorbances in the case with 0.1%(w/v) BSA in place of human IL-18 precursor or human IL-18 were under 0.01. These results mean that in this assay, a value of the difference apparently over 0.01, for example, higher than 0.05, indicates the existence of the objective protein in samples and that from the value, the concentration of the protein can be measured. With regard to this criterion, the enzyme-immunoassay of this Example proved to detect human IL-18 precursor at a concentration not less than about 5 ng/ml, as shown in FIG. 3. FIG. 3 additionally shows that this assay quantitatively detected the precursor at a concentration of about 5 ng/ml to about 100 ng/ml. On the contrary, this assay detected human IL-18 only at a concentration over about 1,000 ng/ml. These facts mean that this assay detects human IL-18 precursor selectively, about 200 times as sensitive as it does human IL-18.

The second antibody used in this Example exhibits an immunoreactivity, as mentioned above, against human IL-18 as well as against human IL-18 precursor. This means that the sensitivity of this assay that is differential to human IL-18 precursor and human IL-18 are due to the deferential immunoreactivity of the first antibody against them. Therefore, the results of this Example indicate that the first antibody is an antibody specific to human IL-18 precursor, whose immunoreactivity against human IL-18 is only at about 0.5% intensity of that against the precursor. In addition, the other monoclonal antibodies obtained in Example 1-3(c) were studied similarly as in this Example. Each antibody exhibited an immunoreactivity against human IL-18 only at about 10% to about 2% or less of that against human IL-18 precursor. Thus the antibodies were also confirmed specific to human IL-18 precursor.

Although not illustrated in detail, other antibodies specific to non-human IL-18 precursor can be prepared with reference to the disclosure by this invention. For example, SEQ ID NO:13 shows the nucleotide sequence encoding mouse IL-18 precursor, with which manipulations corresponding to Examples 1 and 2 can provide desired antibodies and detection methods. In addition, the antibody of this invention, specific to IL-18 precursor, can be beneficially used, for example, in affinity chromatography to purify IL-18 precursor and in elimination or detoxification of IL-18 precursor accumulated *in vivo.*

As described above, the antibody of this invention is specific to IL-18 precursor, exhibiting an immunoreactivity against IL-18 precursor at a higher intensity than against any other substance. The present antibody is feasible for various uses such as detection and purification of IL-18 precursor as well as for pharmaceutical uses. The present antibody having such usefulness can be obtained in desired amounts by the processes of this invention to prepare the present antibody.

This invention, exhibiting remarkable effects as described above, greatly contributes to the art.

### SEQUENCE LISTING

<110> Kabushiki Kaisha Hayashibara Seibutsu Kagaku Kenkyujo
<120> Antibody specific to interleukin 18 precursor
<130>
<160> 13
<150> JP 324,860/99
   <151> 1999-11-16
<210> 1
   <211> 36
   <212> PRT
   <213> Homo sapiens
<400> 1
   Met Ala Ala Glu Pro Val Glu Asp Asn Cys Ile Asn Phe Val Ala Met
   1 5 10 15
   Lys Phe Ile Asp Asn Thr Leu Tyr Phe Ile Ala Glu Asp Asp Glu Asn
   20 25 30
   Leu Glu Ser Asp
   35
<210> 2
   <211> 35
   <212> PRT
   <213> Mus Musculs
<400> 2
   Met Ala Ala Met Ser Glu Asp Ser Cys Val Asn Phe Lys Glu Met Met
   1 5 10 15
   Phe Ile Asp Asn Thr Leu Tyr Phe Ile Pro Glu Glu Asn Gly Asp Leu
   20 25 30
   Glu Ser Asp
   35
<210> 3
   <211> 157
   <212> PRT
   <213> Homo sapiens
<220>
   <221> UNSURE
   <222> (73)
   <223> Xaa is Ile or Thr
<400> 3
   Tyr Phe Gly Lys Leu Glu Ser Lys Leu Ser Val Ile Arg Asn Leu Asn
   1 5 10 15
   Asp Gln Val Leu Phe Ile Asp Gln Gly Asn Arg Pro Leu Phe Glu Asp
   20 25 30
   Met Thr Asp Ser Asp Cys Arg Asp Asn Ala Pro Arg Thr Ile Phe Ile
   35 40 45
   Ile Ser Met Tyr Lys Asp Ser Gln Pro Arg Gly Met Ala Val Thr Ile
   50 55 60
   Ser Val Lys Cys Glu Lys Ile Ser Xaa Leu Ser Cys Glu Asn Lys Ile
   65 70 75 80
   Ile Ser Phe Lys Glu Met Asn Pro Pro Asp Asn Ile Lys Asp Thr Lys
   85 90 95
   Ser Asp Ile Ile Phe Phe Gln Arg Ser Val Pro Gly His Asp Asn Lys
   100 105 110
   Met Gln Phe Glu Ser Ser Ser Tyr Glu Gly Tyr Phe Leu Ala Cys Glu
   115 120 125
   Lys Glu Arg Asp Leu Phe Lys Leu Ile Leu Lys Lys Glu Asp Glu Leu
   130 135 140
   Gly Asp Arg Ser Ile Met Phe Thr Val Gln Asn Glu Asp
   145 150 155
<210> 4
   <211> 193
   <212> PRT
   <213> Homo sapiens
<220>
   <221> PROPEP
   <222> (-36)...(-1)
<220>
   <221> CHAIN
   <222> (1) ... (157)
<220>
   <221> UNSURE
   <222> (73)
   <223> Xaa is Ile or Thr
<400> 4
   Met Ala Ala Glu Pro Val Glu Asp Asn Cys Ile Asn Phe Val Ala Met
   -35 -30 -25
   Lys Phe Ile Asp Asn Thr Leu Tyr Phe Ile Ala Glu Asp Asp Glu Asn
   -20 -15 -10 -5
   Leu Glu Ser Asp Tyr Phe Gly Lys Leu Glu Ser Lys Leu Ser Val Ile
   1 5 10
   Arg Asn Leu Asn Asp Gln Val Leu Phe Ile Asp Gln Gly Asn Arg Pro
   15 20 25
   Leu Phe Glu Asp Met Thr Asp Ser Asp Cys Arg Asp Asn Ala Pro Arg
   30 35 40
   Thr Ile Phe Ile Ile Ser Met Tyr Lys Asp Ser Gln Pro Arg Gly Met
   45 50 55 60
   Ala Val Thr Ile Ser Val Lys Cys Glu Lys Ile Ser Xaa Leu Ser Cys
   65 70 75
   Glu Asn Lys Ile Ile Ser Phe Lys Glu Met Asn Pro Pro Asp Asn Ile
   80 85 90
   Lys Asp Thr Lys Ser Asp Ile Ile Phe Phe Gln Arg Ser Val Pro Gly
   95 100 105
   His Asp Asn Lys Met Gln Phe Glu Ser Ser Ser Tyr Glu Gly Tyr Phe
   110 115 120
   Leu Ala Cys Glu Lys Glu Arg Asp Leu Phe Lys Leu Ile Leu Lys Lys
   125 130 135 140
   Glu Asp Glu Leu Gly Asp Arg Ser Ile Met Phe Thr Val Gln Asn Glu
   145 150 155
   Asp
<210> 5
   <211> 157
   <212> PRT
   <213> Mus musculus
<220>
   <221> UNSURE
   <222> (70)
   <223> Xaa is Met or Thr
<400> 5
   Asn Phe Gly Arg Leu His Cys Thr Thr Ala Val Ile Arg Asn Ile Asn
   1 5 10 15
   Asp Gln Val Leu Phe Val Asp Lys Arg Gln Pro Val Phe Glu Asp Met
   20 25 30
   Thr Asp Ile Asp Gln Ser Ala Ser Glu Pro Gln Thr Arg Leu Ile Ile
   35 40 45
   Tyr Met Tyr Lys Asp Ser Glu Val Arg Gly Leu Ala Val Thr Leu Ser
   50 55 60
   Val Lys Asp Ser Lys Xaa Ser Thr Leu Ser Cys Lys Asn Lys Ile Ile
   65 70 75 80
   Ser Phe Glu Glu Met Asp Pro Pro Glu Asn Ile Asp Asp Ile Gln Ser
   85 90 95
   Asp Leu Ile Phe Phe Gln Lys Arg Val Pro Gly His Asn Lys Met Glu
   100 105 110
   Phe Glu Ser Ser Leu Tyr Glu Gly His Phe Leu Ala Cys Gln Lys Glu
   115 120 125
   Asp Asp Ala Phe Lys Leu Ile Leu Lys Lys Lys Asp Glu Asn Gly Asp
   130 135 140
   Lys Ser Val Met Phe Thr Leu Thr Asn Leu His Gln Ser
   145 150 155
<210> 6
   <211> 192
   <212> PRT
   <213> Mus musculus
<220>
   <221> PROPEP
   <222> (-35)...(-1)
<220>
   <221> CHAIN
   <222> (1)... (157)
<220>
   <221> UNSURE
   <222> (70)
   <223> Xaa is Met or Thr
<400> 6
   Met Ala Ala Met Ser Glu Asp Ser Cys Val Asn Phe Lys Glu Met Met
   -35 -30 -25 -20
   Phe Ile Asp Asn Thr Leu Tyr Phe Ile Pro Glu Glu Asn Gly Asp Leu
   -15 -10 -5
   Glu Ser Asp Asn Phe Gly Arg Leu His Cys Thr Thr Ala Val Ile Arg
   1 5 10
   Asn Ile Asn Asp Gln Val Leu Phe Val Asp Lys Arg Gln Pro Val Phe
   15 20 25
   Glu Asp Met Thr Asp Ile Asp Gln Ser Ala Ser Glu Pro Gln Thr Arg
   30 35 40 45
   Leu Ile Ile Tyr Met Tyr Lys Asp Ser Glu Val Arg Gly Leu Ala Val
   50 55 60
   Thr Leu Ser Val Lys Asp Ser Lys Xaa Ser Thr Leu Ser Cys Lys Asn
   65 70 75
   Lys Ile Ile Ser Phe Glu Glu Met Asp Pro Pro Glu Asn Ile Asp Asp
   80 85 90
   Ile Gln Ser Asp Leu Ile Phe Phe Gln Lys Arg Val Pro Gly His Asn
   95 100 105
   Lys Met Glu Phe Glu Ser Ser Leu Tyr Glu Gly His Phe Leu Ala Cys
   110 115 120 125
   Gln Lys Glu Asp Asp Ala Phe Lys Leu Ile Leu Lys Lys Lys Asp Glu
   130 135 140
   Asn Gly Asp Lys Ser Val Met Phe Thr Leu Thr Asn Leu His Gln Ser
   145 150 155
<210> 7
   <211> 582
   <212> DNA
   <213> Homo sapiens
<220>
   <221> CDS
   <222> (1)...(582)
<220>
   <221> mat peptide
   <222> (109)...(579)
<220>
   <221> UNSURE
   <222> (325) ... (327)
   <223> Xaa is Ile or Thr
<400> 7
   atg gct gct gaa cca gta gaa gac aat tgc atc aac ttt gtg gca atg     48
   Met Ala Ala Glu Pro Val Glu Asp Asn Cys Ile Asn Phe Val Ala Met
       -35                 -30                 -25
   aaa ttt att gac aat acg ctt tac ttt ata gct gaa gat gat gaa aac     96
   Lys Phe Ile Asp Asn Thr Leu Tyr Phe Ile Ala Glu Asp Asp Glu Asn
   -20                 -15                 -10                  -5
   ctg gaa tca gat tac ttt ggc aag ctt gaa tct aaa tta tca gtc ata    144
   Leu Glu Ser Asp Tyr Phe Gly Lys Leu Glu Ser Lys Leu Ser Val Ile
                     1               5                  10
   aga aat ttg aat gac caa gtt ctc ttc att gac caa gga aat cgg cct    192
   Arg Asn Leu Asn Asp Gln Val Leu Phe Ile Asp Gln Gly Asn Arg Pro
            15                  20                  25
   cta ttt gaa gat atg act gat tct gac tgt aga gat aat gca ccc cgg    240
   Leu Phe Glu Asp Met Thr Asp Ser Asp Cys Arg Asp Asn Ala Pro Arg
       30                  35                  40
   acc ata ttt att ata agt atg tat aaa gat agc cag cct aga ggt atg    288
   Thr Ile Phe Ile Ile Ser Met Tyr Lys Asp Ser Gln Pro Arg Gly Met
   45                  50                  55                  60
   gct gta act atc tct gtg aag tgt gag aaa att tca ayt ctc tcc tgt    336
   Ala Val Thr Ile Ser Val Lys Cys Glu Lys Ile Ser Xaa Leu Ser Cys
                   65                  70                  75
   gag aac aaa att att tcc ttt aag gaa atg aat cct cct gat aac atc    384
   Glu Asn Lys Ile Ile Ser Phe Lys Glu Met Asn Pro Pro Asp Asn Ile
               80                  85                  90
   aag gat aca aaa agt gac atc ata ttc ttt cag aga agt gtc cca gga    432
   Lys Asp Thr Lys Ser Asp Ile Ile Phe Phe Gln Arg Ser Val Pro Gly
           95                 100                 105
   cat gat aat aag atg caa ttt gaa tct tca tca tac gaa gga tac ttt    480
   His Asp Asn Lys Met Gln Phe Glu Ser Ser Ser Tyr Glu Gly Tyr Phe
       110                 115                 120
   cta gct tgt gaa aaa gag aga gac ctt ttt aaa ctc att ttg aaa aaa    528
   Leu Ala Cys Glu Lys Glu Arg Asp Leu Phe Lys Leu Ile Leu Lys Lys
   125                 130                 135                 140
   gag gat gaa ttg ggg gat aga tct ata atg ttc act gtt caa aac gaa    576
   Glu Asp Glu Leu Gly Asp Arg Ser Ile Met Phe Thr Val Gln Asn Glu
                   145                 150                 155
   gac tag                                                            582
   Asp
<210> 8
   <211> 27
   <212> DNA
   <213> Artificial sequence
<220>
<223> Designed oligonucleotide as a sense primer for PCR to amplify a DNA fragment containing a coding sequence for a part of propeptide sequence of human IL-18 precursor
<400> 8
   agagatctgc tgctgaacca gtagaag 27
<210> 9
   <211> 45
   <212> DNA
   <213> Artificial sequence
<220>
   <223> Designed oligonucleotide as an antisense primer for PCR to amplify a DNA fragment containing a coding sequence for a part of propeptide sequence of human IL-18 precursor
<400> 9
   tcaagcttag tgatggtgat ggtgatgatc tgattccagg ttttc 45
<210> 10
   <211> 49
   <212> DNA
   <213> Artificial sequence
<220>
<223> Designed oligonucleotide as a sense primer for PCR to amplify a DNA fragment containing a coding sequence for human IL-18 precursor
<400> 10
   ggtcgggatc tgtacgacga tgacgataag atggctgctg aaccagtag 49
<210> 11
   <211> 30
   <212> DNA
   <213> Artificial sequence
<220>
   <223> Designed oligonucleotide as an antisense primer for PCR to amplify a DNA fragment containing a coding sequence for human IL-18 precursor
<400> 11
   tgctcgagtt agtcttcgtt ttgaacagtg 30
<210> 12
   <211> 47
   <212> DNA
   <213> Artificial sequence
<220>
   <223> Designed oligonucleotide as a sense primer for PCR to amplify a DNA fragment containing a coding sequence for human IL-18 precursor
<400> 12
   ggctagcatg actggtggac agcaaatggg tcgggatctg tacgacg 47
<210> 13
   <211> 579
   <212> DNA
   <213> Mus musculus
<220>
   <221> CDS
   <222> (1)... (579)
<220>
   <221> mat peptide
   <222> (106) ... (576)
<220>
   <221> UNSURE
   <222> (313)... (315)
   <223> Xaa is Met or Thr
<400> 13
   atg gct gcc atg tca gaa gac tct tgc gtc aac ttc aag gaa atg atg   48
   Met Ala Ala Met Ser Glu Asp Ser Cys Val Asn Phe Lys Glu Met Met
   -35                 -30                 -25                 -20
   ttt att gac aac acg ctt tac ttt ata cct gaa gaa aat gga gac ctg   96
   Phe Ile Asp Asn Thr Leu Tyr Phe Ile Pro Glu Glu Asn Gly Asp Leu
                   -15                 -10                 -5
   gaa tca gac aac ttt ggc cga ctt cac tgt aca acc gca gta ata cgg   144
   Glu Ser Asp Asn Phe Gly Arg Leu His Cys Thr Thr Ala Val Ile Arg
                 1               5                  10
   aat ata aat gac caa gtt ctc ttc gtt gac aaa aga cag cct gtg ttc   192
   Asn Ile Asn Asp Gln Val Leu Phe Val Asp Lys Arg Gln Pro Val Phe
        15                  20                  25
   gag gat atg act gat att gat caa agt gcc agt gaa ccc cag acc aga   240
   Glu Asp Met Thr Asp Ile Asp Gln Ser Ala Ser Glu Pro Gln Thr Arg
    30                  35                  40                  45
   ctg ata ata tac atg tac aaa gac agt gaa gta aga gga ctg gct gtg   288
   Leu Ile Ile Tyr Met Tyr Lys Asp Ser Glu Val Arg Gly Leu Ala Val
                    50                  55                  60
   acc ctc tct gtg aag gat agt aaa ayg tct acc ctc tcc tgt aag aac   336
   Thr Leu Ser Val Lys Asp Ser Lys Xaa Ser Thr Leu Ser Cys Lys Asn
                65                  70                  75
   aag atc att tcc ttt gag gaa atg gat cca cct gaa aat att gat gat   384
   Lys Ile Ile Ser Phe Glu Glu Met Asp Pro Pro Glu Asn Ile Asp Asp
            80                  85                  90
   ata caa agt gat ctc ata ttc ttt cag aaa cgt gtt cca gga cac aac   432
   Ile Gln Ser Asp Leu Ile Phe Phe Gln Lys Arg Val Pro Gly His Asn
        95                 100                 105
   aag atg gag ttt gaa tct tca ctg tat gaa gga cac ttt ctt gct tgc   480
   Lys Met Glu Phe Glu Ser Ser Leu Tyr Glu Gly His Phe Leu Ala Cys
   110                 115                 120                 125
   caa aag gaa gat gat gct ttc aaa ctc att ctg aaa aaa aag gat gaa   528
   Gln Lys Glu Asp Asp Ala Phe Lys Leu Ile Leu Lys Lys Lys Asp Glu
                   130                 135                 140
   aat ggg gat aaa tct gta atg ttc act ctc act aac tta cat caa agt   576
   Asn Gly Asp Lys Ser Val Met Phe Thr Leu Thr Asn Leu His Gln Ser
                145                 150                 155
   tag                                                               579

### SEQUENCE LISTING

<110> Kabushiki Kaisha Hayashibara Seibutsu Kagaku Kenkyujo
<120> Antibody specific to interleukin 18 precursor
<130>
   <160> 13
<150> JP 324,860/99
   <151> 1999-11-16
<210> 1
   <211> 36
   <212> PRT
   <213> Homo sapiens
<400> 1
   Met Ala Ala Glu Pro Val Glu Asp Asn Cys Ile Asn Phe Val Ala Met
   1 5 10 15
   Lys Phe Ile Asp Asn Thr Leu Tyr Phe Ile Ala Glu Asp Asp Glu Asn
   20 25 30
   Leu Glu Ser Asp
   35
<210> 2
   <211> 35
   <212> PRT
   <213> Mus Musculs
<400> 2
   Met Ala Ala Met Ser Glu Asp Ser Cys Val Asn Phe Lys Glu Met Met
   1 5 10 15
   Phe Ile Asp Asn Thr Leu Tyr Phe Ile Pro Glu Glu Asn Gly Asp Leu
   20 25 30
   Glu Ser Asp
   35
<210> 3
   <211> 157
   <212> PRT
   <213> Homo sapiens
<220>
   <221> UNSURE
   <222> (73)
   <223> Xaa is Ile or Thr
<400> 3
   Tyr Phe Gly Lys Leu Glu Ser Lys Leu Ser Val Ile Arg Asn Leu Asn
   1 5 10 15
   Asp Gln Val Leu Phe Ile Asp Gln Gly Asn Arg Pro Leu Phe Glu Asp
   20 25 30
   Met Thr Asp Ser Asp Cys Arg Asp Asn Ala Pro Arg Thr Ile Phe Ile
   35 40 45
   Ile Ser Met Tyr Lys Asp Ser Gln Pro Arg Gly Met Ala Val Thr Ile
   50 55 60
   Ser Val Lys Cys Glu Lys Ile Ser Xaa Leu Ser Cys Glu Asn Lys Ile
   65 70 75 80
   Ile Ser Phe Lys Glu Met Asn Pro Pro Asp Asn Ile Lys Asp Thr Lys
   85 90 95
   Ser Asp Ile Ile Phe Phe Gln Arg Ser Val Pro Gly His Asp Asn Lys
   100 105 110
   Met Gln Phe Glu Ser Ser Ser Tyr Glu Gly Tyr Phe Leu Ala Cys Glu
   115 120 125
   Lys Glu Arg Asp Leu Phe Lys Leu Ile Leu Lys Lys Glu Asp Glu Leu
   130 135 140
   Gly Asp Arg Ser Ile Met Phe Thr Val Gln Asn Glu Asp
   145 150 155
<210> 4
   <211> 193
   <212> PRT
   <213> Homo sapiens
<220>
   <221> PROPEP
   <222> (-36)...(-1)
<220>
   <221> CHAIN
   <222> (1) ... (157)
<220>
   <221> UNSURE
   <222> (73)
   <223> Xaa is Ile or Thr
<400> 4
   Met Ala Ala Glu Pro Val Glu Asp Asn Cys Ile Asn Phe Val Ala Met
   -35 -30 -25
   Lys Phe Ile Asp Asn Thr Leu Tyr Phe Ile Ala Glu Asp Asp Glu Asn
   -20 -15 -10 -5
   Leu Glu Ser Asp Tyr Phe Gly Lys Leu Glu Ser Lys Leu Ser Val Ile
   1 5 10
   Arg Asn Leu Asn Asp Gln Val Leu Phe Ile Asp Gln Gly Asn Arg Pro
   15 20 25
   Leu Phe Glu Asp Met Thr Asp Ser Asp Cys Arg Asp Asn Ala Pro Arg
   30 35 40
   Thr Ile Phe Ile Ile Ser Met Tyr Lys Asp Ser Gln Pro Arg Gly Met
   45 50 55 60
   Ala Val Thr Ile Ser Val Lys Cys Glu Lys Ile Ser Xaa Leu Ser Cys
   65 70 75
   Glu Asn Lys Ile Ile Ser Phe Lys Glu Met Asn Pro Pro Asp Asn Ile
   80 85 90
   Lys Asp Thr Lys Ser Asp Ile Ile Phe Phe Gln Arg Ser Val Pro Gly
   95 100 105
   His Asp Asn Lys Met Gln Phe Glu Ser Ser Ser Tyr Glu Gly Tyr Phe
   110 115 120
   Leu Ala Cys Glu Lys Glu Arg Asp Leu Phe Lys Leu Ile Leu Lys Lys
   125 130 135 140
   Glu Asp Glu Leu Gly Asp Arg Ser Ile Met Phe Thr Val Gln Asn Glu
   145 150 155
   Asp
<210> 5
   <211> 157
   <212> PRT
   <213> Mus musculus
<220>
   <221> UNSURE
   <222> (70)
   <223> Xaa is Met or Thr
<400> 5
   Asn Phe Gly Arg Leu His Cys Thr Thr Ala Val Ile Arg Asn Ile Asn
   1               5                   10                  15
   Asp Gln Val Leu Phe Val Asp Lys Arg Gln Pro Val Phe Glu Asp Met
               20                  25                  30
   Thr Asp Ile Asp Gln Ser Ala Ser Glu Pro Gln Thr Arg Leu Ile Ile
           35                  40                  45
   Tyr Met Tyr Lys Asp Ser Glu Val Arg Gly Leu Ala Val Thr Leu Ser
       50                  55                  60
   Val Lys Asp Ser Lys Xaa Ser Thr Leu Ser Cys Lys Asn Lys Ile Ile
   65                  70                  75                  80
   Ser Phe Glu Glu Met Asp Pro Pro Glu Asn Ile Asp Asp Ile Gln Ser
                   85                  90                  95
   Asp Leu Ile Phe Phe Gln Lys Arg Val Pro Gly His Asn Lys Met Glu
               100                 105                 110
   Phe Glu Ser Ser Leu Tyr Glu Gly His Phe Leu Ala Cys Gln Lys Glu
           115                 120                 125
   Asp Asp Ala Phe Lys Leu Ile Leu Lys Lys Lys Asp Glu Asn Gly Asp
       130                 135                 140
   Lys Ser Val Met Phe Thr Leu Thr Asn Leu His Gln Ser
   145                 150                 155
<210> 6
   <211> 192
   <212> PRT
   <213> Mus musculus
<220>
   <221> PROPEP
   <222> (-35)...(-1)
<220>
   <221> CHAIN
   <222> (1)... (157)
<220>
   <221> UNSURE
   <222> (70)
   <223> Xaa is Met or Thr
<400> 6
   Met Ala Ala Met Ser Glu Asp Ser Cys Val Asn Phe Lys Glu Met Met
   -35                 -30                 -25                 -20
   Phe Ile Asp Asn Thr Leu Tyr Phe Ile Pro Glu Glu Asn Gly Asp Leu
                   -15                 -10                  -5
   Glu Ser Asp Asn Phe Gly Arg Leu His Cys Thr Thr Ala Val Ile Arg
                 1               5                  10
   Asn Ile Asn Asp Gln Val Leu Phe Val Asp Lys Arg Gln Pro Val Phe
        15                  20                  25
   Glu Asp Met Thr Asp Ile Asp Gln Ser Ala Ser Glu Pro Gln Thr Arg
    30                  35                  40                  45
   Leu Ile Ile Tyr Met Tyr Lys Asp Ser Glu Val Arg Gly Leu Ala Val
                    50                  55                  60
   Thr Leu Ser Val Lys Asp Ser Lys Xaa Ser Thr Leu Ser Cys Lys Asn
                65                  70                  75
   Lys Ile Ile Ser Phe Glu Glu Met Asp Pro Pro Glu Asn Ile Asp Asp
            80                  85                  90
   Ile Gln Ser Asp Leu Ile Phe Phe Gln Lys Arg Val Pro Gly His Asn
        95                 100                 105
   Lys Met Glu Phe Glu Ser Ser Leu Tyr Glu Gly His Phe Leu Ala Cys
   110                 115                 120                 125
   Gln Lys Glu Asp Asp Ala Phe Lys Leu Ile Leu Lys Lys Lys Asp Glu
                   130                 135                 140
   Asn Gly Asp Lys Ser Val Met Phe Thr Leu Thr Asn Leu His Gln Ser
               145                 150                 155
<210> 7
   <211> 582
   <212> DNA
   <213> Homo sapiens
<220>
   <221> CDS
   <222> (1) ... (582)
<220>
   <221> mat peptide
   <222> (109) ... (579)
<220>
   <221> UNSURE
   <222> (325) ... (327)
   <223> Xaa is Ile or Thr
<400> 7
   atg gct gct gaa cca gta gaa gac aat tgc atc aac ttt gtg gca atg    48
   Met Ala Ala Glu Pro Val Glu Asp Asn Cys Ile Asn Phe Val Ala Met
       -35                 -30                 -25
   aaa ttt att gac aat acg ctt tac ttt ata gct gaa gat gat gaa aac    96
   Lys Phe Ile Asp Asn Thr Leu Tyr Phe Ile Ala Glu Asp Asp Glu Asn
   -20                 -15                 -10                 -5
   ctg gaa tca gat tac ttt ggc aag ctt gaa tct aaa tta tca gtc ata   144
   Leu Glu Ser Asp Tyr Phe Gly Lys Leu Glu Ser Lys Leu Ser Val Ile
                       1               5                   10
   aga aat ttg aat gac caa gtt ctc ttc att gac caa gga aat cgg cct   192
   Arg Asn Leu Asn Asp Gln Val Leu Phe Ile Asp Gln Gly Asn Arg Pro
           15                  20                  25
   cta ttt gaa gat atg act gat tct gac tgt aga gat aat gca ccc cgg   240
   Leu Phe Glu Asp Met Thr Asp Ser Asp Cys Arg Asp Asn Ala Pro Arg
       30                  35                  40
   acc ata ttt att ata agt atg tat aaa gat agc cag cct aga ggt atg   288
   Thr Ile Phe Ile Ile Ser Met Tyr Lys Asp Ser Gln Pro Arg Gly Met
   45                  50                  55                  60
   gct gta act atc tct gtg aag tgt gag aaa att tca ayt ctc tcc tgt   336
   Ala Val Thr Ile Ser Val Lys Cys Glu Lys Ile Ser Xaa Leu Ser Cys
                   65                  70                  75
   gag aac aaa att att tcc ttt aag gaa atg aat cct cct gat aac atc   384
   Glu Asn Lys Ile Ile Ser Phe Lys Glu Met Asn Pro Pro Asp Asn Ile
               80                  85                  90
   aag gat aca aaa agt gac atc ata ttc ttt cag aga agt gtc cca gga   432
   Lys Asp Thr Lys Ser Asp Ile Ile Phe Phe Gln Arg Ser Val Pro Gly
           95                  100                 105
   cat gat aat aag atg caa ttt gaa tct tca tca tac gaa gga tac ttt   480
   His Asp Asn Lys Met Gln Phe Glu Ser Ser Ser Tyr Glu Gly Tyr Phe
       110                 115                 120
   cta gct tgt gaa aaa gag aga gac ctt ttt aaa ctc att ttg aaa aaa   528
   Leu Ala Cys Glu Lys Glu Arg Asp Leu Phe Lys Leu Ile Leu Lys Lys
   125                 130                 135                 140
   gag gat gaa ttg ggg gat aga tct ata atg ttc act gtt caa aac gaa   576
   Glu Asp Glu Leu Gly Asp Arg Ser Ile Met Phe Thr Val Gln Asn Glu
                   145                 150                 155
   gac tag                                                           582
   Asp
<210> 8
   <211> 27
   <212> DNA
   <213> Artificial sequence
<220>
   <223> Designed oligonucleotide as a sense primer for PCR to amplify a DNA fragment containing a coding sequence for a part of propeptide sequence of human IL-18 precursor
<400> 8
   agagatctgc tgctgaacca gtagaag 27
<210> 9
   <211> 45
   <212> DNA
   <213> Artificial sequence
<220>
   <223> Designed oligonucleotide as an antisense primer for PCR to amplify a DNA fragment containing a coding sequence for a part of propeptide sequence of human IL-18 precursor
<400> 9
   tcaagcttag tgatggtgat ggtgatgatc tgattccagg ttttc 45
<210> 10
   <211> 49
   <212> DNA
   <213> Artificial sequence
<220>
<223> Designed oligonucleotide as a sense primer for PCR to amplify a DNA fragment containing a coding sequence for human IL-18 precursor
<400> 10
   ggtcgggatc tgtacgacga tgacgataag atggctgctg aaccagtag 49
<210> 11
   <211> 30
   <212> DNA
   <213> Artificial sequence
<220>
<223> Designed oligonucleotide as an antisense primer for PCR to amplify a DNA fragment containing a coding sequence for human IL-18 precursor
<400> 11
   tgctcgagtt agtcttcgtt ttgaacagtg 30
<210> 12
   <211> 47
   <212> DNA
   <213> Artificial sequence
<220>
<223> Designed oligonucleotide as a sense primer for PCR to amplify a DNA fragment containing a coding sequence for human IL-18 precursor
<400> 12
   ggctagcatg actggtggac agcaaatggg tcgggatctg tacgacg 47
<210> 13
   <211> 579
   <212> DNA
   <213> Mus musculus
<220>
   <221> CDS
   <222> (1) ... (579)
<220>
   <221> mat peptide
   <222> (106) ... (576)
<220>
   <221> UNSURE
   <222> (313)... (315)
   <223> Xaa is Met or Thr
<400> 13
   atg gct gcc atg tca gaa gac tct tgc gtc aac ttc aag gaa atg atg   48
   Met Ala Ala Met Ser Glu Asp Ser Cys Val Asn Phe Lys Glu Met Met
   -35                 -30                 -25                 -20
   ttt att gac aac acg ctt tac ttt ata cct gaa gaa aat gga gac ctg   96
   Phe Ile Asp Asn Thr Leu Tyr Phe Ile Pro Glu Glu Asn Gly Asp Leu
                   -15                 -10                 -5
   gaa tca gac aac ttt ggc cga ctt cac tgt aca acc gca gta ata cgg   144
   Glu Ser Asp Asn Phe Gly Arg Leu His Cys Thr Thr Ala Val Ile Arg
                1               5                  10
   aat ata aat gac caa gtt ctc ttc gtt gac aaa aga cag cct gtg ttc   192
   Asn Ile Asn Asp Gln Val Leu Phe Val Asp Lys Arg Gln Pro Val Phe
        15                  20                  25
   gag gat atg act gat att gat caa agt gcc agt gaa ccc cag acc aga   240
   Glu Asp Met Thr Asp Ile Asp Gln Ser Ala Ser Glu Pro Gln Thr Arg
    30                  35                  40                  45
   ctg ata ata tac atg tac aaa gac agt gaa gta aga gga ctg gct gtg   288
   Leu Ile Ile Tyr Met Tyr Lys Asp Ser Glu Val Arg Gly Leu Ala Val
                    50                  55                  60
   acc ctc tct gtg aag gat agt aaa ayg tct acc ctc tcc tgt aag aac   336
   Thr Leu Ser Val Lys Asp Ser Lys Xaa Ser Thr Leu Ser Cys Lys Asn
                65                  70                  75
   aag atc att tcc ttt gag gaa atg gat cca cct gaa aat att gat gat   384
   Lys Ile Ile Ser Phe Glu Glu Met Asp Pro Pro Glu Asn Ile Asp Asp
            80                  85                  90
   ata caa agt gat ctc ata ttc ttt cag aaa cgt gtt cca gga cac aac   432
   Ile Gln Ser Asp Leu Ile Phe Phe Gln Lys Arg Val Pro Gly His Asn
        95                 100                 105
   aag atg gag ttt gaa tct tca ctg tat gaa gga cac ttt ctt gct tgc   480
   Lys Met Glu Phe Glu Ser Ser Leu Tyr Glu Gly His Phe Leu Ala Cys
   110                 115                 120                 125
   caa aag gaa gat gat gct ttc aaa ctc att ctg aaa aaa aag gat gaa   528
   Gln Lys Glu Asp Asp Ala Phe Lys Leu Ile Leu Lys Lys Lys Asp Glu
                   130                 135                 140
   aat ggg gat aaa tct gta atg ttc act ctc act aac tta cat caa agt   576
   Asn Gly Asp Lys Ser Val Met Phe Thr Leu Thr Asn Leu His Gln Ser
               145                 150                 155
   tag                                                               579

## Claims

1. An antibody specific to an interleukin 18 precursor, said antibody being obtainable from a warm-blooded animal which has been immunized with a polypeptide comprising a part or the whole of the propeptide amino acid sequence of the precursor.

2. An antibody according to claim 1, whose immunoreactivity against mature interleukin 18 is at most ten percent intensity of that against the precursor.

3. An antibody according to any preceding claim, wherein the precursor is of a primate or rodent origin.

4. An antibody according to any preceding claim, wherein the precursor comprises the propeptide sequence shown in SEQ ID No:1 or 2.

5. An antibody according to any preceding claim, which is in the form of a polyclonal or monoclonal antibody.

6. An isolated cell capable of producing an antibody as defined in any preceding claim.

7. A cell according to claim 7, which is in the form of a hybridoma.

8. An immunoassay kit comprising an antibody as defined in any of claims 1-5.

9. A process to prepare an antibody, which comprises the steps of immunizing a non-human warm-blooded animal with a polypeptide comprising a part or the whole of the propeptide amino acid sequence of an interleukin 18 precursor and collecting an antibody according to claim 1 from a body fluid of the immunized animal.

10. A process according to claim 9, wherein the polypeptide to immunize the non-human warm-blooded animal comprises a part or the whole of the propeptide amino acid sequence shown in SEQ ID NO:1 or 2 as a part of the precursor sequences.

11. A process according to claim 9 or claim 10, wherein the antibody is collected by one or more purification techniques selected from salting out, dialysis, filtration, concentration, centrifugation, seperatory sedimentation, gel filtration chromatography, ion-exchange chromatography, high performance liquid chromatography, affinity chromatography, gel electrophoresis, and isoelectric focusing electrophoresis.

12. A process to prepare an antibody, which comprises the steps of culturing *in vivo* with the exception of human beings or *in vitro* an isolated cell capable of producing an antibody as defined in any of claims 1-5 and collecting the antibody from the resulting product of the *in vivo* with the exception of human beings or *in vitro* culture.

13. A process according to claim 12, wherein the isolated cell is a hybridoma.

14. A process according to claim 12 or claim 13, wherein the antibody is collected by one or more purification techniques' selected from salting out, dialysis, filtration concentration, centrifugation, seperatory sedimentation, gel filtration chromatography, ion-exchange chromatography, high performance liquid chromatography, affinity chromatography, gel electrophoresis, and isoelectric focusing electrophoresis.

15. A method to detect an interleukin 18 precursor, which comprises the steps of contacting a sample with an antibody as defined in any of claims 1-5 to effect an immunoreaction and detecting the precursor on the basis of the immunoreaction.

16. A method according to claim 15, which is performed in accordance with an immunoassay selected from an enzyme-immunoassay, radioimmunoassay, and fluoroimmunoassay.

17. A method according to claim 15 or claim 16, wherein the sample is a biological sample collected from a living body.

18. A detection method effected by performing a method as defined in any of claims 15-17 in combination with a detection method for mature interleukin 18 using an antibody against the mature interleukin 18.

19. A method according to claim 15 or claim 18, which is a diagnostic method for a disease relating to mature interleukin 18 or interleukin 18 precursor.

20. A method to purify an interleukin 18 precursor, which comprises the steps of contacting a mixture containing the precursor and an impurity with an antibody as defined in any of claims 1-5 to adsorb the precursor on the antibody, and desorbing the precursor from the antibody.

21. A method according to claim 20, wherein the antibody is immobilized on a water-insoluble carrier.

22. A pharmaceutical composition comprising an antibody as defined in any of claims 1-5.

23. A pharmaceutical composition according to claim 22, which additionally comprises a physiologically acceptable carrier.

24. A pharmaceutical composition according to claim 22 or claim 23, which is a remedial, alleviative, or preventive agent for a disease such as an autoimmune disease relating to mature interleukin 18 or interleukin 18 precursor.

25. Use of antibody as defined in any of claims 1-5 in the manufacture of a medicament effective in treating a disease relating to mature interleukin 18 or interleukin 18 precursor, such as an autoimmune disease.

## Patentansprüche

1. Antikörper, spezifisch für einen Interleukin-18 Vorläufer, wobei besagter Antikörper von einem warmblütigen Tier erhalten werden kann, das mit einem Polypeptid immunisiert wurde, welches einen Teil oder die gesamte Aminosäuresequenz des Propeptids des Vorläufers umfasst.

2. Antikörper gemäß Anspruch 1, dessen Immunoreaktivität gegenüber dem ausgereiften Interleukin-18 höchstens zehn Prozent von der Intensität der Immunoreaktivität gegenüber dem Vorläufer beträgt.

3. Antikörper gemäß einem der vorangegangenen Ansprüche, wobei der Vorläufer von Primaten oder von Nagern stammt.

4. Antikörper gemäß einem der vorangegangenen Ansprüche, wobei der Vorläufer die in SEQ ID No: 1 oder 2 gezeigte Sequenz des Propeptids umfasst.

5. Antikörper gemäß einem der vorangegangenen Ansprüche, der in Form eines polyklonalen oder monoklonalen Antikörpers vorliegt.

6. Isolierte Zelle mit der Fähigkeit, einen in einem der vorangegangenen Ansprüche definierten Antikörper zu produzieren.

7. Zelle gemäß Anspruch 6, die in Form einer Hybridomazelle vorliegt.

8. Immuntest-Ausstattung, welche einen in den Ansprüchen 1-5 definierten Antikörper umfasst.

9. Verfahren zur Bereitstellung eines Antikörpers, das die Arbeitsschritte umfasst: Immunisierung eines nicht-menschlichen, warmblütigen Tieres mit einem einen Teil oder die gesamte Aminosäuresequenz des Propeptids eines Interleukin-18 Vorläufers umfassenden Polypeptids und Entnahme eines Antikörpers gemäß Anspruch 1 aus einer Körperflüssigkeit des immunisierten Tieres.

10. Verfahren gemäß Anspruch 9, wobei das Polypeptid zur Immunisierung des nicht-menschlichen, warmblütigen Tieres einen Teil oder die gesamte Aminosäuresequenz des in SEQ ID No:1 oder 2 gezeigten Propeptids als Teil der Sequenzen des Vorläufers umfasst.

11. Verfahren gemäß Anspruch 9 oder 10, wobei der Antikörper durch ein oder mehrere Reinigungsverfahren angereichert wird, ausgewählt aus: Kristallisation, Dialyse, Filtration, Eindampfen, Zentrifugieren, fraktionierte Fällung, Gel-Filtrations-Chromatographie, Ionenaustauschchromatographie, Hochleistungsflüssigkeitschromatographie, Affinitätschromatographie, Gel-Elektrophorese und Elektrophorese mit isoelektrischer Fokussierung.

12. Verfahren zur Bereitstellung eines Antikörpers, das die Arbeitsschritte umfasst: In vivo-Kultivierung, mit Ausnahme von menschlichen Wesen, oder *in vitro-*Kultivierung einer isolierten Zelle mit der Fähigkeit, einen in den Ansprüchen 1-5 definierten Antikörper zu produzieren, und Entnahme des Antikörpers aus dem entstandenen Produkt der *in vivo*-Kultur, mit Ausnahme von menschlichen Wesen, oder in vitro-Kultur.

13. Verfahren gemäß Anspruch 12, wobei die isolierte Zelle eine Hybridomazelle ist.

14. Verfahren gemäß Anspruch 12 oder 13, wobei der Antikörper durch ein oder mehrere Reinigungsverfahren angereichert wird, ausgewählt aus: Kristallisation, Dialyse, Filtration, Eindampfen, Zentrifugieren, fraktionierte Fällung, Gel-Filtrations-Chromatographie, Ionenaustauschchromatographie, Hochleistungsflüssigkeitschromatographie, Affinitätschromatographie, Gel-Elektrophorese und Elektrophorese mit isoelektrischer Fokussierung

15. Verfahren zur Bestimmung eines Interleukin-18 Vorläufers, das die Arbeitsschritte umfasst: Inkontaktbringen einer Probe mit einem in einem der Ansprüche 1-5 definierten Antikörper, um eine Immunreaktion auszulösen, und Bestimmung des Vorläufers anhand der Immunreaktion.

16. Verfahren gemäß Anspruch 15, das in Form eines Immuntest, ausgewählt aus einem Enzymimmuntest, einem Radioimmuntest und einem Fluorimmuntest durchgeführt wird.

17. Verfahren gemäß Anspruch 15 oder 16, wobei die Probe eine biologische Probe ist, die einem lebenden Körper entnommen wird.

18. Bestimmungsmethode, die durch die Durchführung eines der in einem der Ansprüche 15-17 definierten Verfahren in Kombination mit einer Bestimmungsmethode für ausgereiftes Interleukin-18, wobei ein Antikörper gegen das ausgereifte Interleukin-18 eingesetzt wird, erfolgt.

19. Verfahren gemäß Anspruch 15 oder 18, das ein diagnostisches Verfahren für eine Krankheit im Zusammenhang mit ausgereiftem Interleukin-18 oder einem Interleukin-18 Vorläufer ist.

20. Verfahren, zur Reinigung eines Interleukin-18 Vorläufers, das die Arbeitsschritte umfasst: Inkontaktbringen einer den Vorläufer und eine Verunreinigung enthaltenden Mischung mit einem in einem der Ansprüche 1-5 definierten Antikörper, um den Vorläufer auf dem Antikörper zu adsorbieren, und Desorbieren des Vorläufers von dem Antikörper.

21. Verfahren gemäß Anspruch 20, wobei der Antikörper auf einem wasserunlöslichen Träger fixiert ist.

22. Pharmazeutische Zusammensetzung, die einen in einem der Ansprüche 1-5 definierten Antikörper umfasst.

23. Pharmazeutische Zusammensetzung gemäß Anspruch 22, die zusätzlich einen physiologisch verträglichen Träger umfasst.

24. Pharmazeutische Zusammensetzung gemäß Anspruch 22 oder 23, die ein heilendes, linderndes oder vorbeugendes Mittel für Krankheiten, wie Autoimmunerkrankungen im Zusammenhang mit ausgereiftem Interleukin-18 oder einem Interleukin-18 Vorläufer ist.

25. Verwendung eines in einem der Ansprüche 1-5 definierten Antikörpers zur Herstellung eines Medikaments, das wirksam ist bei der Behandlung einer Krankheit im Zusammenhang mit ausgereiftem Interleukin-18 oder einem Interleukin-18 Vorläufer, wie z.B. einer Autoimmunerkrankung.

## Revendications

1. Anticorps spécifique d'un précurseur d'interleukine 18, ledit anticorps pouvant être obtenu à partir d'un animal à sang chaud qui a été immunisé avec un polypeptide comprenant une partie ou la totalité de la séquence d'aminoacides de propeptide du précurseur.

2. Anticorps suivant la revendication 1, dont l'immunoréactivité vis-à-vis de l'interleukine 18 mature est d'une intensité d'au plus dix pour cent de celle contre le précurseur.

3. Anticorps suivant l'une quelconque des revendications précédentes, ledit précurseur étant issu d'un primate ou d'un rongeur.

4. Anticorps suivant l'une quelconque des revendications précédentes, ledit précurseur comprenant la séquence de propeptide représentée dans la SEQ ID N° 1 ou 2.

5. Anticorps suivant l'une quelconque des revendications précédentes, qui est sous forme d'un anticorps polyclonal ou monoclonal.

6. Cellule isolée capable de produire un anticorps tel que défini dans l'une quelconque des revendications précédentes.

7. Cellule suivant la revendication 7, qui est sous forme d'un hybridome.

8. Kit d'analyse immunologique comprenant un anticorps tel que défini dans l'une quelconque des revendications 1 à 5.

9. Procédé pour la préparation d'un anticorps, qui comprend les étapes consistant à immuniser un animal non humain à sang chaud avec un polypeptide comprenant une partie ou la totalité de la séquence d'aminoacides de propeptide d'un précurseur d'interleukine 18 et à recueillir un anticorps suivant la revendication 1 à partir d'un fluide corporel de l'animal immunisé.

10. Procédé suivant la revendication 9, dans lequel le polypeptide pour immuniser l'animal non humain à sang chaud comprend une partie ou la totalité de la séquence d'aminoacides de propeptide représentée dans la SEQ ID N° 1 ou 2 en tant qu'une partie des séquences de précurseur.

11. Procédé suivant la revendication 9 ou la revendication 10, dans lequel l'anticorps est recueilli par une ou plusieurs techniques de purification choisies entre le relargage, la dialyse, la filtration, la concentration, la centrifugation, la sédimentation séparatrice, la chromatographie de filtration sur gel, la chromatographie d'échange d'ions, la chromatographie en phase liquide à hautes performances, la chromatographie d'affinité, l'électrophorèse sur gel et l'électrophorèse par focalisation isoélectrique.

12. Procédé de préparation d'un anticorps, qui comprend les étapes consistant à cultiver *in vivo*, à l'exception d'êtres humains, ou *in vitro* une cellule isolée capable de produire un anticorps tel que défini dans l'une quelconque des revendications 1 à 5, et à recueillir l'anticorps à partir du produit résultant de la culture *in vivo,* à l'exception d'êtres humains, ou *in vitro.*

13. Procédé suivant la revendication 12, dans lequel la cellule isolée est un hybridome.

14. Procédé suivant la revendication 12 ou la revendication 13, dans lequel l'anticorps est recueilli par une ou plusieurs techniques de purification choisies entre le relargage, la dialyse, la filtration, la concentration, la centrifugation, la sédimentation séparatrice, la chromatographie de filtration sur gel, la chromatographie d'échange d'ions, la chromatographie en phase liquide à hautes performances, la chromatographie d'affinité, l'électrophorèse sur gel et l'électrophorèse par focalisation isoélectrique.

15. Méthode pour détecter un précurseur d'interleukine 18, qui comprend les étapes consistant à mettre en contact un échantillon avec un anticorps tel que défini dans l'une quelconque des revendications 1 à 5 pour effectuer une immunoréaction, et à détecter le précurseur sur la base de l'immunoréaction.

16. Méthode suivant la revendication 15, qui est mise en oeuvre par une analyse immunologique choisie entre une analyse immunoenzymatique, une analyse radioimmunologique et une analyse fluoro-immunologique.

17. Méthode suivant la revendication 15 ou la revendication 16, dans laquelle l'échantillon est un échantillon biologique recueilli à partir d'un organisme vivant.

18. Méthode de détection effectuée en mettant en oeuvre une méthode telle que définie dans l'une quelconque des revendications 15 à 17, en association avec une méthode de détection de l'interleukine 18 mature utilisant un anticorps contre l'interleukine 18 mature.

19. Méthode suivant la revendication 15 ou la revendication 18, qui est une méthode de diagnostic d'une maladie en rapport avec l'interleukine 18 mature ou un précurseur d'interleukine 18.

20. Méthode pour purifier un précurseur d'interleukine 18, qui comprend les étapes consistant à mettre en contact un mélange contenant le précurseur et une impureté avec un anticorps tel que défini dans l'une quelconque des revendications 1 à 5 pour adsorber le précurseur sur l'anticorps, et à désorber le précurseur de l'anticorps.

21. Méthode suivant la revendication 20, dans laquelle l'anticorps est immobilisé sur un support insoluble dans l'eau.

22. Composition pharmaceutique comprenant un anticorps tel que défini dans l'une quelconque des revendications 1 à 5.

23. Composition pharmaceutique suivant la revendication 22, qui comprend en outre un support physiologiquement acceptable.

24. Composition pharmaceutique suivant la revendication 22 ou la revendication 23, qui est un agent curatif, agent atténuant ou agent préventif pour une maladie telle qu'une maladie auto-immune en rapport avec l'interleukine 18 mature ou un précurseur d'interleukine 18.

25. Utilisation d'un anticorps tel que défini dans l'une quelconque des revendications 1 à 5, dans la production d'un médicament efficace dans le traitement d'une maladie en rapport avec l'interleukine 18 mature ou un précurseur d'interleukine 18, telle qu'une maladie auto-immune.
